# EUROPEAN PATENT APPLICATION

(11) **EP 4 458 289 A1**
(43) Date of publication of application: **06.11.2024**
(21) Application number: 22914792.1
(22) Date of filing: 27.12.2022
(51) Int. Cl.: A61B 17/3207

(54) **RESECTION APPARATUS**

(30) Priority: 31.12.2021 CN 202111677854; 31.12.2021 CN 202111672557; 31.12.2021 CN 202111678798; 31.12.2021 CN 202111677042; 31.12.2021 CN 202111677839; 31.12.2021 CN 202111677863
(71) Applicant: Lifetech Scientific (Shenzhen) Co., Ltd., Shenzhen, Guangdong 518063 (CN)
(72) Inventor: LI, Wen, Shenzhen, Guangdong 518063 (CN); ZHANG, Xiong, Shenzhen, Guangdong 518063 (CN); ZHENG, Di, Shenzhen, Guangdong 518063 (CN); FANG, Jinfeng, Shenzhen, Guangdong 518063 (CN); WANG, Zeyang, Shenzhen, Guangdong 518063 (CN)
(74) Representative: Michalski Hüttermann & Partner Patentanwälte mbB
(86) International application number: PCT/CN2022/142344
(87) International publication number: WO 2023/125539

(57) **Abstract**

A resection apparatus includes a handle body, a catheter communicating with the handle body, and a cutter head assembly disposed at a distal end of the catheter. A steering assembly is disposed on the catheter and includes a first steering point and a second steering point which are disposed on the catheter, and a steering connector for connecting the first steering point and the second steering point. A control assembly for controlling the steering assembly is disposed on the handle body. The bending radius of the catheter at the distal end is adjusted by means of the steering assembly, so that the catheter is shifted towards a predetermined direction; the orientation of the cutter head assembly is controlled, so as to reduce a risk of scraping and perforating a blood vessel, and the ideal blood vessel diameter can be obtained by means of multiple resections.

## Description

### Technical Field

The present disclosure relates in general to the technical field of medical instruments, and more particularly relates to a resection apparatus.

### Background

An incidence of vascular diseases has risen sharply with continued increase of degree of aging of the population in China and changes in a diet structure. Peripheral arterial disease is one of the vascular diseases. A main cause of the peripheral arterial disease is atherosclerosis, which is often manifested as ischemic changes of limbs, celiac artery, carotid artery, renal artery, etc. Treatment methods mainly include basic treatment based on drug treatment, including open surgical operation treatment represented by a classical surgical bypass surgery and recently developed endovascular interventional treatment.

Although the surgical bypass surgery has a relatively excellent long-term patency rate, an endovascular interventional treatment technique has been gradually accepted by clinicians and patients due to its advantages of minimal intervention, safety, effectiveness and repeatability. Herein, peripheral atherectomy is mainly for femoral, popliteal and uinferior genicular artery stenosis or occlusion.

However, an existing resection system for the peripheral atherectomy has a risk that the catheter is prone to scraping a vascular wall when being pushed. In clinical applications, the resection system may be adjusted in a bending radius of a tube body at a distal end, so that an atherectomy cutter head is shifted towards a certain angle, and may more thoroughly clear a blocking plaque in a blood vessel. However, in a steering state, the distal end of the catheter may form a certain angle with the blood vessel, resulting in that the catheter is not flush with the blood vessel in an axial direction, and thus is prone to scraping and even perforating the blood vessel.

Therefore, there is a need for a new technical means to solve the above problems in the prior art.

### Summary

An objective of the present disclosure is to solve at least the problem of a resection apparatus having a cutter head assembly that is prone to scraping an inner wall of a blood vessel when the catheter is steered.

In one embodiment, a resection apparatus is disclosed. The resection apparatus includes a handle body, a catheter communicating with the handle body, and a cutter head assembly disposed at the distal end of the catheter, where a steering assembly is disposed on the catheter and includes a first steering point and a second steering point which are disposed on the catheter, and a steering connector for connecting the first steering point and the second steering point, and a control assembly for controlling the steering assembly is disposed on the handle body.

Through the resection apparatus, a bending radius of the catheter at the distal end is adjusted by means of the steering assembly, so that the catheter is shifted toward a predetermined direction; the orientation of the cutter head assembly is controlled, so as to reduce the risk of scraping and perforating a blood vessel, and an ideal blood vessel diameter may be obtained by means of multiple resections.

In addition, the resection apparatus disclosed by embodiments also has the following additional technical features:
According to some embodiments hereof, the steering connector is fixedly connected to the first steering point, and movablely connected to the second steering point; the distance between the first steering point and the distal end of the catheter is smaller than that between the second steering point and the distal end of the catheter; and the first steering point is disposed on the other side opposite to the second steering point.

According to some embodiments hereof, the steering connector includes a steering control wire; a control wire cavity through which the steering control wire extends is formed in the catheter; the second steering point is disposed at the distal end of the control wire cavity; and the steering control wire extends through the second steering point, rotates around the catheter, and then is fixedly connected to the first steering point.

According to some embodiments hereof, the control assembly includes a control seat disposed on the handle body and a wire takeup device movably connected to the control seat; and the wire takeup device is connected to the steering control wire and used for drawing the steering control wire to adjust the posture of the catheter.

According to some embodiments hereof, a torque shaft connected to the cutter head assembly is disposed in the catheter, and a driving mechanism for driving the torque shaft to rotate is disposed in the handle body.

According to some embodiments hereof, the cutter head assembly includes a limiting seat connected to the catheter and an atherectomy cutter head disposed on the limiting seat; the atherectomy cutter head is fixedly connected to one end of the torque shaft; the torque shaft drives the atherectomy cutter head to be rotatably connected to the limiting seat; and a protective sleeve covering a side surface of the atherectomy cutter head is disposed on the limiting seat.

According to some embodiments hereof, the catheter includes an inner tube and an outer tube disposed on an outer side of the inner tube; the inner tube surrounds an outer side of the torque shaft; the torque shaft and inner tube are in clearance fit; and a braided layer is disposed between the inner tube and the outer tube.

According to some embodiments hereof, a discharge cavity channel connected to the catheter is formed in a handle body, and communicates with the catheter and external space of the handle body.

According to some embodiments hereof, the torque shaft extends through the discharge cavity channel, and is connected to the driving mechanism; and the driving mechanism includes a power source, a driving transmission assembly for connecting the power source and the torque shaft, and a power supply electrically connected to the power source.

According to some embodiments hereof, the discharge cavity channel includes a main cavity communicating with the catheter and a waste discharge tube disposed on a side surface of the main cavity; and the torque shaft extends through the discharge cavity channel.

### Brief Description of the Drawings

FIG. 1 is a schematic structural diagram of a resection apparatus in an embodiment I;
FIG. 2 is a schematic diagram of an interior structure of a handle body in the embodiment I;
FIG. 3 is a schematic structural diagram of a driving transmission assembly in the embodiment I;
FIG. 4 is a schematic structural diagram of a cutter head assembly in the embodiment I;
FIG. 5 is a schematic structural diagram of a catheter in the embodiment I;
FIG. 6 is a schematic structural diagram of a steering assembly in the embodiment I;
FIG. 7 is a schematic structural diagram of a bent catheter in the embodiment I;
FIG. 8 is a schematic diagram of an overall structure of a control assembly in the embodiment I;
FIG. 9 is a cross-sectional view of the control assembly in the embodiment I;
FIG. 10 is a side view of a cross section of the control assembly in the embodiment I;
FIG. 11 is a schematic diagram of an overall structure when an adjustment module is in low-speed cutting in the embodiment I;
FIG. 12 is a schematic diagram of an overall structure when an adjustment module is in high-speed cutting in the embodiment I;
FIG. 13 is a schematic diagram of a partial structure of the adjustment module in low-speed cutting in the embodiment I;
FIG. 14 is a schematic diagram of a partial structure of the adjustment module in high-speed cutting in the embodiment I;
FIG. 15 is a schematic structural diagram of a speed control key in the embodiment I;
FIG. 16 is a schematic diagram of an overall structure of a disassembly connection portion in the embodiment I;
FIG. 17 is an exploded view of the handle body in the embodiment I;
FIG. 18 is a schematic diagram of a partial structure of a clamping assembly in the embodiment I;
FIG. 19 is a schematic diagram of a connection structure of the handle body and a handle upper cover in the embodiment I;
FIG. 20 is a schematic structural diagram of connection of the handle body and a driving mechanism in the embodiment I;
FIG. 21 is a schematic diagram of a partial structure of a clamping assembly in the embodiment I;
FIG. 22 is a schematic structural diagram of a catheter and a steering assembly in an embodiment II;
FIG. 23 is a schematic diagram of an overall structure of a handle body in an embodiment III;
FIG. 24 is a schematic diagram of an interior structure of a control assembly in the embodiment III;
FIG. 25 is a schematic diagram of an overall structure of a handle body in an embodiment IV;
FIG. 26 is a schematic diagram of an interior structure of a control assembly in the embodiment IV;
FIG. 27 is a schematic diagram of a partial structure of the control assembly in the embodiment IV;
FIG. 28 is a schematic diagram of a perspective structure of a handle body in an embodiment V;
FIG. 29 is a schematic diagram of an interior structure of the handle body in the embodiment V;
FIG. 30 is an exploded view of the handle body in the embodiment V;
FIG. 31 is a schematic diagram of a partial structure of a catheter in the embodiment V;
FIG. 32 is an enlarged view of the part A in FIG. 29 in the embodiment V; and
FIG. 33 is an enlarged view of a part B in FIG. 29 in the embodiment V.

### Detailed Description

Hereinafter, exemplary embodiments of the present disclosure will be described in detail with reference to the accompanying drawings. While exemplary implementations of the present disclosure are shown in the drawings, it should be understood that the present disclosure may be embodied in various forms and should not be limited to the implementations set forth herein. Rather, these implementations are provided so that the present disclosure will be thoroughly understood, and will fully convey the scope of the present disclosure to those skilled in the art.

It is to be understood that the terms used herein is for the purpose of describing particular exemplary implementations only and is not intended to be limiting. As used herein, the singular forms "a", "an" and "the" are intended to include the plural forms as well, unless the context clearly indicates otherwise. The terms "comprise", "include", "contain" and "have" are inclusive and therefore indicate the existence of the stated features, steps, operations, elements and/or components, but do not exclude the existence or addition of one or more other features, steps, operations, elements, components, and/or combinations thereof. The method steps, processes, and operations described here shall not be construed as necessarily requiring that they be performed in a particular sequence described or illustrated here, unless the sequence for performance is expressly indicated. It should also be understood that additional or alternative steps may be used.

Although the terms first, second, third, etc. can be used in the text to describe a plurality of elements, components, regions, layers, and/or sections, these elements, components, regions, layers, and/or sections should not be limited by these terms. These terms can only be used to distinguish one element, component, region, layer, or segment from another region, layer, or segment. Unless explicitly stated, terms such as "first", "second", and other numerical terms used in the text do not imply sequence or order.

For ease of description, spatial relative relationship terms, such as, "internal", "external", "inner side", "outer side", "under", "below", "on", "above", may be used herein to describe the relationship of one element or feature relative to another element or feature as illustrated in the figures. These spatially relative terms are intended to encompass different orientations of the device in use or operation in addition to the orientation depicted in the figures. For example, if the device in the figures is turned over, elements described as "below" or "beneath" other elements or features would then be oriented "on" or "above" the other elements or features. Therefore, the example term "under" can include up and down orientations. The apparatus can be additionally oriented (rotated by 90 degrees or in other directions) and the spatial relative relationship terms used herein is interpreted accordingly.

For the ease of description, the following description uses the terms "proximal" and "distal", where the "proximal" refers to positions close to the operator, and the "distal" refers to positions far away from the operator. The phrase "axial direction" in the application should be understood to indicate a direction in which an interventional device is pushed in and out, and a direction perpendicular to the "axial direction" is defined as the "radial direction".

### Embodiment I

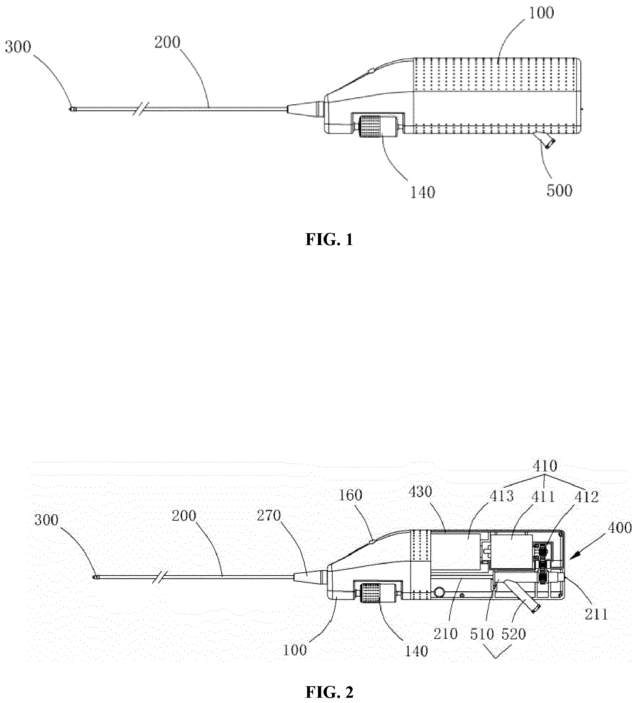

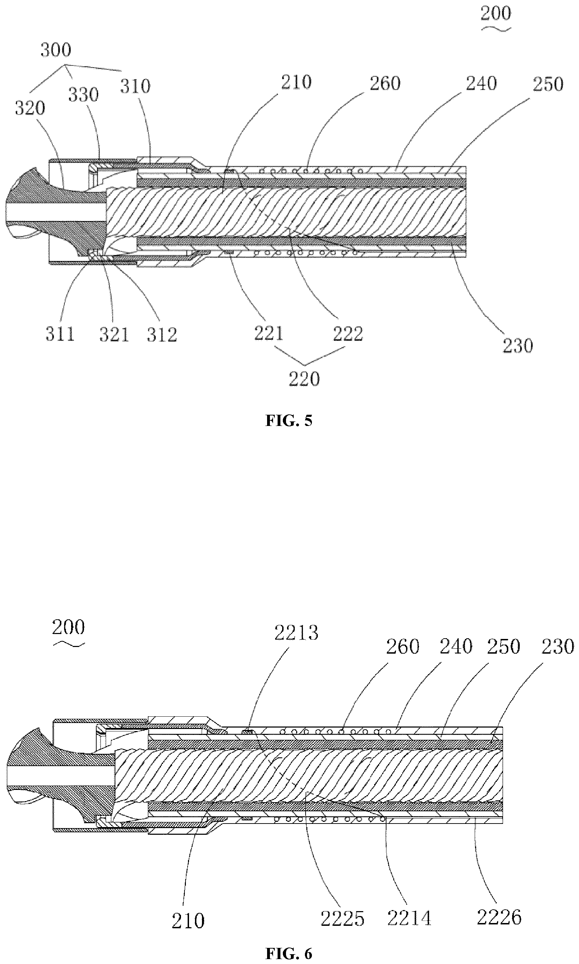

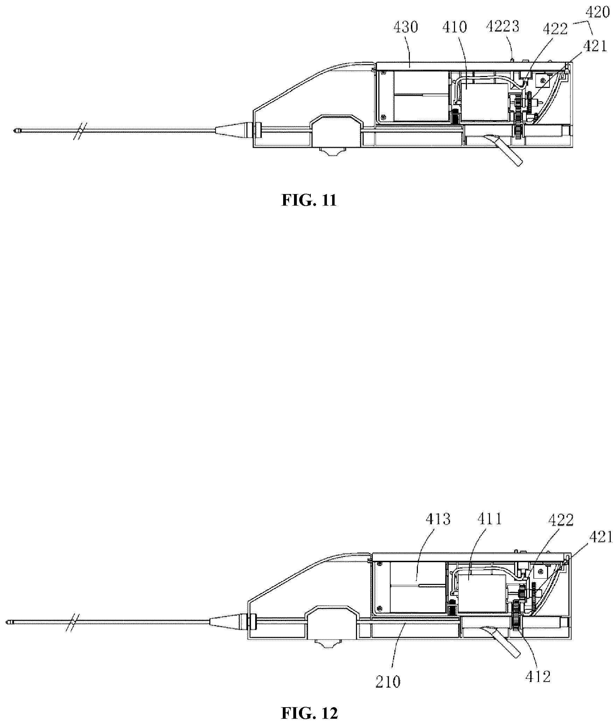

Embodiment I of the present disclosure provides a resection apparatus, as shown in FIGS. 1 to 3, including a handle body 100, a catheter 200 communicating with the handle body 100, and a cutter head assembly 300 disposed at a distal end of the catheter 200, where a torque shaft 210 connected to the cutter head assembly 300 is disposed in the catheter 200. As shown in FIG. 5, a steering assembly 220 for steering a distal end portion of the catheter 200 is disposed on the catheter 200; and a driving mechanism 400 is detachably connected to the handle body 100. As shown in FIG. 11, the driving mechanism 400 includes a driving module 410 for driving the torque shaft 210 to rotate, and an adjustment module 420 for controlling the driving module 410 to adjust torque of the torque shaft 210.

In the present disclosure, a bending radius of the catheter 200 at the distal end is adjusted by means of the steering assembly 220, so that the catheter 200 is shifted toward a predetermined direction; the orientation of the cutter head assembly 300 is controlled, so as to reduce a risk of scraping and perforating a blood vessel, and an ideal blood vessel diameter may be obtained by means of multiple resections. Meanwhile, the adjustment module 420 for adjusting the torque of the torque shaft 210 is disposed, so that a plaque resection apparatus may more smoothly resect a plaque by adjusting the torque of the torque shaft 210 when resecting a hard plaque. In the other aspect, by arranging the driving mechanism 400 to be detachably connected to the handle body 100, the driving mechanism 400 may be recycled to reduce the cost of the plaque resection apparatus.

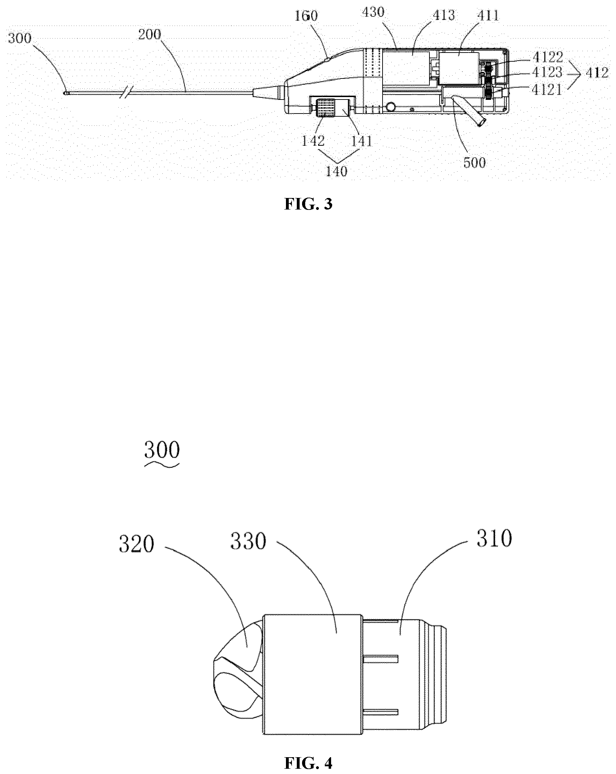

Additionally, in the embodiment shown in FIG. 4, the cutter head assembly 300 includes a limiting seat 310 connected to the catheter 200 and an atherectomy cutter head 320 disposed on the limiting seat 310; the atherectomy cutter head 320 is fixedly connected to one end of the torque shaft 210; the torque shaft 210 drives the atherectomy cutter head 320 to be rotatably connected to the limiting seat 310; and a protective sleeve 330 covering a side surface of the atherectomy cutter head 320 is disposed on the limiting seat 310.

As shown in FIG. 5, the catheter 200 includes an inner tube 230 and an outer tube 240 disposed on an outer side of the inner tube 230; the inner tube 230 surrounds an outer side of the torque shaft 210; the torque shaft 210 and the inner tube 230 are in clearance fit; and the torque shaft 210 extends through an entire inner cavity of the inner tube 230.

In addition, a braided layer 250 is disposed between the inner tube 230 and the outer tube 240, where the braided layer 250 is specifically a braided net which is braided by nickel-titanium wires. The inner tube 230 and the outer tube 240 are connected by means of the braided layer 250, so that toughness of the catheter 200 is strengthened. When the catheter 200 extends into a branch blood vessel or passes through a bent portion of the blood vessel, the catheter 200 will not have a local sunken or bent phenomenon, which can maintain the integrity of a lumen of the catheter 200 and the passing performance of the resected plaque, so that the plaque cannot block the bent part of the catheter 200.

Additionally, in one embodiment, the atherectomy cutter head 320 is fixed to an end part of the torque shaft 210 by means of welding, and the torque shaft 210 rotates to drive the atherectomy cutter head 320 to rotate. A cutter head clamping groove 321 is formed in the atherectomy cutter head 320; and a limiting boss 311 is disposed at the distal end of the limiting seat 310, and is in clamping fit with the cutter head clamping groove 321, so that axial and radial limitations between the atherectomy cutter head 320 and the limiting seat 310 are achieved.

The atherectomy cutter head 320, the limiting seat 310 and the protective sleeve 330 are all made of stainless steel. Meanwhile, an elastic sheet 312 having elasticity is disposed at a distal end of the limiting seat 310; and the limiting boss 311 is disposed on the elastic sheet 312. As the cutter head assembly 300 is made of a hard metal material as a whole, the assembly process of the atherectomy cutter head 320 and the limiting seat 310 is complicated. In this embodiment, the elastic sheet 312 is disposed at the distal end of the limiting seat 310, and can elastically deform during assembly due to its elasticity, so that the limiting boss 311 can be smoothly clamped into the cutter head clamping groove 321. The protective sleeve 330 covers an outer side of the limiting seat 310, and besides, grips an outer surface of the limiting seat 310. Therefore, after the protective sleeve 330 is assembled, the elastic sheet 312 is limited between the atherectomy cutter head 320 and the protective sleeve 310, so as to complete the clamping fit between the limiting boss 312 and the cutter head clamping groove 321, and then achieve the axial and radial limitations between the atherectomy cutter head 320 and the limiting seat 310.

The catheter 200 sequentially includes the inner tube 230, the braided layer 250 and the outer tube 240 from inside to outside; and the inner tube 230, the braided layer 250 and the outer tube 240 form an integrated body by means of hot melting. In addition, a spring tube 260 is disposed at the distal end of the catheter 200; the spring tube 260 surrounds the outer side of the braided layer 250, or the braided layer 250 surrounds the outer side of the spring tube 260; and the spring tube 260 is axially fixed with the braided layer 250 by means of welding. In this embodiment, the spring tube 260 surrounds the outer side of the braided layer 250. By arranging the spring tube 260 at the distal end of the catheter 200, compliance and resilience of the distal end of the catheter 200 are enhanced. In other embodiments, the spring tube 260 can further be embedded into the outer tube 240.

In the present embodiment, due to good bending resistance of the braided layer 250, the spring tube 260 has good torque resistance. Therefore, the braided layer 250 is disposed in the catheter 200, and the structure of arranging the spring tube 260 at the distal end of the catheter 200 is used, which may make the distal end of the catheter 200 have both bending resistance and torque resistance. During steering of the catheter 200, through cooperation of the braided layer 250 and the spring tube 260, local sunken deformation of the catheter 200 due to bending is avoided, and the deformation of the lumen of the catheter 200 can be maintained in the case where the catheter 200 is bent, so as to ensure that the gap between the catheter 200 and the torque shaft 210 is sufficient for the plaque or a thrombus to pass through, and then ensure the smooth operation of a surgery. Meanwhile, good vascular passing performance may also be maintained, making the catheter 200 more smoothly enter a complicatedly bent blood vessel.

The protective sleeve 330 and the outer tube 240 are fixed by means of hot melting. The outer tube 240 is a TPU tube or a Pebax tube. In this embodiment, the outer tube 240 is made of a Pebax material.

In combination with FIG. 2, a buffer sleeve 270 is disposed on the catheter 200; the catheter 200 is connected to the handle body 100 by means of the buffer sleeve 270; the buffer sleeve 270 is fitted to and connected to a shell of the handle body 100 by means of clamping; and the buffer sleeve 270 is made of a TPU material, or other rubber or plastic materials. The buffer sleeve 270 is disposed to be soft, which may eliminate vibration of the catheter 200 during atherectomy, and the vibration transmitted to the handle body 100 will be significantly weakened, thereby providing conditions for a fine operation by an operator.

In addition, in combination with FIGS. 11 and 12, the driving mechanism 400 further includes a driving shell 430, a power source 411 and a driving transmission assembly 412 for connecting the power source 411 and the torque shaft 210. The adjustment module 420 includes an adjustment transmission assembly 421 connected to the driving transmission assembly 412, and a shifting assembly 422; the shifting assembly 422 is used for controlling a fitting relationship between the adjustment transmission assembly 421 and the driving transmission assembly 412, to adjust the torque of the torque shaft 210; and a power supply assembly 413 for driving the power source 411 is disposed in the driving shell 430.

Additionally, in one embodiment, the power source 411 is a motor disposed inside the driving shell 430; the power supply assembly 413 is a battery disposed inside the driving shell 430; and the battery is electrically connected to the motor. The power supply assembly 413 supplies power to the power source 411; the power source 411 drives the torque shaft 210 to rotate by means of the driving transmission assembly 412; and the rotation of the torque shaft 210 causes the atherectomy cutter head 320 disposed at the distal end to rotate, thereby resecting the plaque in the blood vessel. A switch 160 is also disposed on the handle body 100, and a working state of the motor is controlled by the switch 160.

In other embodiments, the power supply assembly 413 connected to an external power supply and electrically connected to the power source 411 can further be disposed on the driving shell 430. The external power supply is connected by means of the power supply assembly 413, when needed to drive the power source 411 to work, and the power supply assembly 413 includes a power socket disposed on the driving shell 430. By means of a form of arranging the power socket, difficulty and cost of sterilization and disinfection of the handle body 100 and internal components can be lowered, and weight and transportation difficulty of the whole resection apparatus may be reduced.

In addition, in combination with FIG. 2, a discharge cavity channel 500 connected to the catheter 200 is formed in the handle body 100; the discharge cavity channel 500 communicates with the catheter 200 and external space of the handle body 100; and the torque shaft 210 extends through the discharge cavity channel 500, and is connected to the driving mechanism 400. The discharge cavity channel 500 includes a main cavity 510 communicating with the catheter 200, and a waste discharge tube 520 disposed on a side surface of the main cavity 510; the torque shaft 210 extends through the main cavity 510, and communicates with the external space of the handle body 100; and a wire guide cavity 211 extending through the torque shaft 210 is disposed inside the torque shaft 210.

In addition, the torque shaft 210 has a spiral arrangement. When the plaque resection apparatus works, the motor drives the torque shaft 210 to rotate. The torque shaft 210 can bring plaque tissue back out of the catheter 200 during rotation due to its spiral arrangement.

The wire guide cavity 211 inside the torque shaft 210 is used for extending through a guide wire. The guide wire is used for extending into the blood vessel or a branch blood vessel before the catheter 200 extends into the blood vessel or moves to establish an approach path of the catheter 200, so that the catheter 200 can move along the approach path established by the guide wire.

A proximal end of the outer tube 240 is bonded and fixed to the main cavity 510. The plaque tissue resected by the atherectomy cutter head 320 passes through the gap between the torque shaft 210 and the inner tube 230, is discharged from the catheter 200 to the discharge cavity channel 500 with the rotation of the torque shaft 210, is temporarily accommodated by the main cavity 510 of the discharge cavity channel 500, and finally is discharged by means of the waste discharge tube 520. The waste discharge tube 520 is disposed at a proximal end close to the main cavity 510, and is inclined in a plaque resection direction, so as to facilitate discharge of the plaque.

In this embodiment, as shown in FIGS. 5 to 7, the control assembly 140 for controlling the steering assembly 220 is disposed on the handle body 100; and the steering assembly 220 includes at least two steering points 221 and a steering connector 222 for connecting the steering points 221. In combination with FIG. 3, the control assembly 140 includes a control seat 141 disposed on the handle body 100 and a wire takeup device 142 movably connected to the control seat 141; and the wire takeup device 142 is used for connecting the steering connector 222 and drawing the steering connector 222 to adjust the posture of the catheter 200.

The steering points 221 include a first steering point 2213 and a second steering point 2214 which are disposed on the catheter 200; the steering connector 222 is fixedly connected to the first steering point 2213, and movablely connected to the second steering point 2214; the distance between the first steering point 2213 and the distal end of the catheter 200 is smaller than that between the second steering point 2214 and the distal end of the catheter 200; and the first steering point 2213 is disposed on the other side opposite to the second steering point 2214.

The steering connector 222 includes a steering control wire 2225. A control wire cavity for the steering control wire 2225 passing therethrough is formed in the catheter 200. The second steering point 2214 is disposed at a distal end of the control wire cavity 2226; and the steering control wire 2225 extends through the second steering point 2214, winds around the catheter 200, and then is fixedly connected to the first steering point 2213. In this embodiment, in order to make the orientation of the cutter head assembly 300 be the same as that of the catheter 200, the circumferential angle between the first steering point 2213 and the second steering point 2214 is 180 degrees.

In one embodiment, the control wire cavity 2226 is formed in the catheter 200, and the control wire cavity 2226 can be formed inside the outer tube 240 or the inner tube 230. The first steering point 2213 is a fixed point, which is fixedly disposed on the braided layer 250 and fixedly connected to the steering control wire 2225, and the second steering point 2214 is an end point of a distal end of the control wire cavity 2226.

In this embodiment, the first steering point 2213 is a fixed ring fixed on the braided layer 250 by means of welding; and the fixed ring tightly presses and fixes the steering control wire 2225 on the braided layer 250, or the steering control wire 2225 is fixed with the fixed ring by means of welding. By arranging the first steering point 2213 as a ring, multi-phase connection of the steering control wire 2225 is satisfied, which is convenient for angle adjustment and fixation of the steering control wire.

One end of the steering control wire 2225 is connected to the wire takeup device 142, and the other end of the steering control wire 2225 first extends out of the control wire cavity 2226, and then is fixed on the first steering point 2213 after spirally rotating by 180 degrees along a gap between the inner tube 230 and the braided layer 250. In addtition, the rotation angle of the steering control wire 2225 can also be 170 degrees or 190 degrees. As long as the second steering point 2214 is relatively disposed on an opposite side of the first steering point 2213, it can be ensured that the orientation of the cutter head assembly 300 is substantially the same as that of the catheter 200 after steering.

In addition, the spiral portion of the steering control wire 2225, with one end extending beyond the second steering point 2214 and the other end fixed to the first steering point 2213, has an axial length that is one to three times as long as the outside diameter of the outer tube 240. If the axial length between the first steering point 2213 and the second steering point 2214 is too long, the steering effect will be poor. If the axial length between the first steering point 2213 and the second steering point 2214 is too short, stress generated by steering is too large, which is unfavorable for operations. Therefore, in this embodiment, the spiral portion of the steering control wire 2225, with one end extending beyond the second steering point 2214 and the other end fixed to the first steering point 2213, has an axial length that is two times as long as the outside diameter of the outer tube 240

In addition, in this embodiment, by adjusting the wire takeup device 142, the steering control wire 2225 is controlled to move in the control wire cavity 2226 or the inner tube 230. As the steering control wire 2225 spirally rotates 180 degrees along the inner tube 230 at the distal end of the catheter 200, the steering control wire 2225 has both axial and radial forces on the distal end of the catheter 200 when the steering control wire 2225 is controlled to shrink, so that axial and radial displacements of the cutter head assembly 300 can be controlled; and as a result, S-shaped steering of the cutter head assembly 300 may be achieved. As the steering control wire 2225 winds around the catheter 200 by 180 degrees, the cutter head assembly 300 and the catheter 200 can keep in the same axial direction, and the risk of the cutter head assembly 300 scraping a vascular wall may be reduced. The cutter head assembly 300 resects the plaque in the blood vessel before and after steering, which can make the blood vessel obtain a larger lumen.

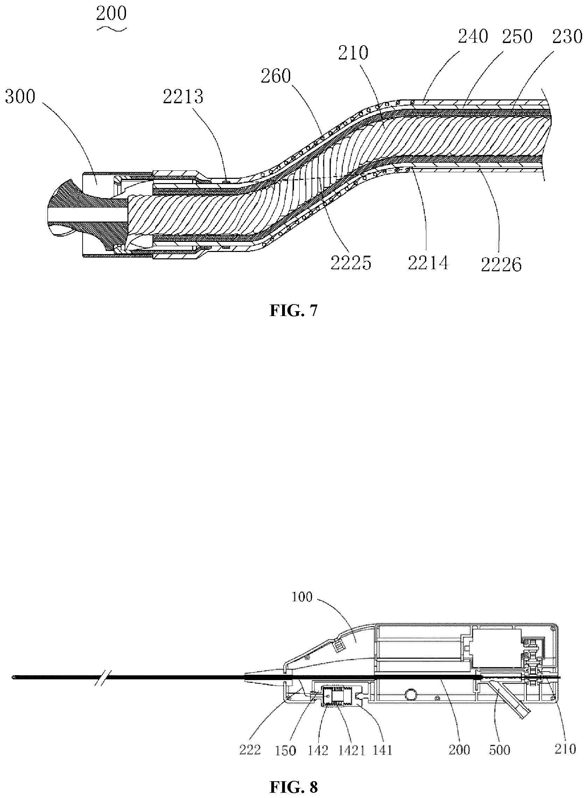

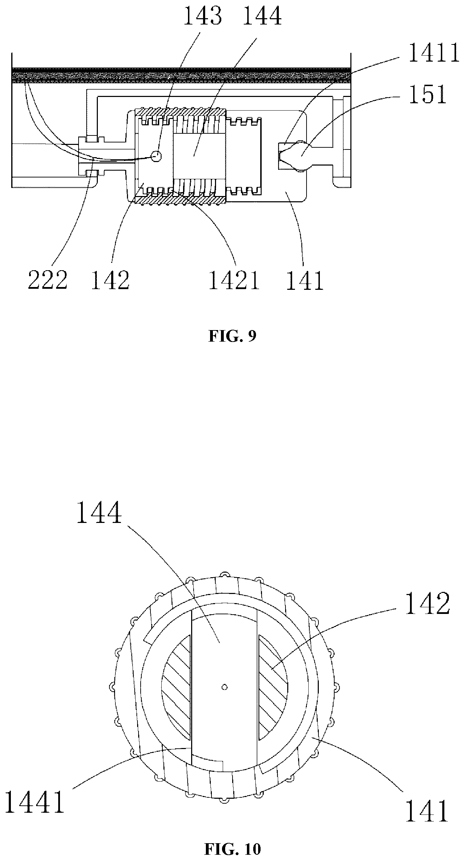

As shown in FIGS. 8 to 10, in this embodiment, the control assembly includes a control seat disposed on the handle body and the wire takeup device movably connected to the control seat; and the wire takeup device includes a locking piece 1421 for self locking.

A control groove 150 is formed in the handle body 100; the control assembly 140 is disposed inside the control groove 150. An ejector rod 151 is disposed at one end of the control groove 150; an ejection groove 1411 is formed in one end, facing the ejector rod 151, of the control seat 141; and the other end of the control seat 141 is rotatably connected to the other end of the control groove 150. Therefore, the control seat 141 is axially fixed in the control groove 150 by means of coordination of the ejection groove 1411 and the ejector rod 151, and the control seat 141 can be rotatably connected to the handle body 100 by means of coordination of the ejection groove 1411 and the ejector rod 151.

The wire takeup device 142 is disposed inside the control seat 141, and a clamping portion 143 used for clamping the steering control wire 2225 is disposed on the wire takeup device 142. A locking piece 1421 includes the internal threads formed in an inner wall of the control seat 141 and external threads formed in an outer wall of the wire takeup device 142; the wire takeup device 142 and the control seat 141 are in threaded connection; and the internal threads in the control seat and the external threads in the wire takeup device are in self-locking threaded fit. After a doctor loosens the wire takeup device, the wire takeup device cannot move relative to the control seat due to the relative self-locking of the internal threads and the external threads, thereby achieving self-locking of the wire takeup device.

A sliding rod 144 is disposed in the control seat 141; the sliding rod 144 is disposed in an axial direction of the control seat 141; the wire takeup device 142 is slidably connected to the sliding rod 144; and a rotation limiting surface 1441 is disposed between the sliding rod 144 and the wire takeup device 142, so that the wire takeup device 142 can translate in an axial direction of the sliding rod 144, and cannot rotate relative to the sliding rod 144, that is, a screw linkage relationship is formed.

The wire takeup device 142 can slip on the sliding rod 144 and move relative to the control seat 141 under linkage of a threaded fit when the control seat 141 is rotated; and the wire takeup device 142 clamps the steering control wire 2225, and the steering control wire 2225 follows the wire takeup device 142 to move relative to the handle body 100, thereby controlling the catheter 200 for a bending action.

In another embodiment as shown in FIGS. 11 to 15, the driving transmission assembly 412 includes a first transmission piece 4121 connected to the torque shaft 210, a second transmission piece 4122 connected to the power source 411, and a third transmission piece 4123 disposed on the driving shell 430. The third transmission piece 4123 is connected between the first transmission piece 4121 and the second transmission piece 4122.

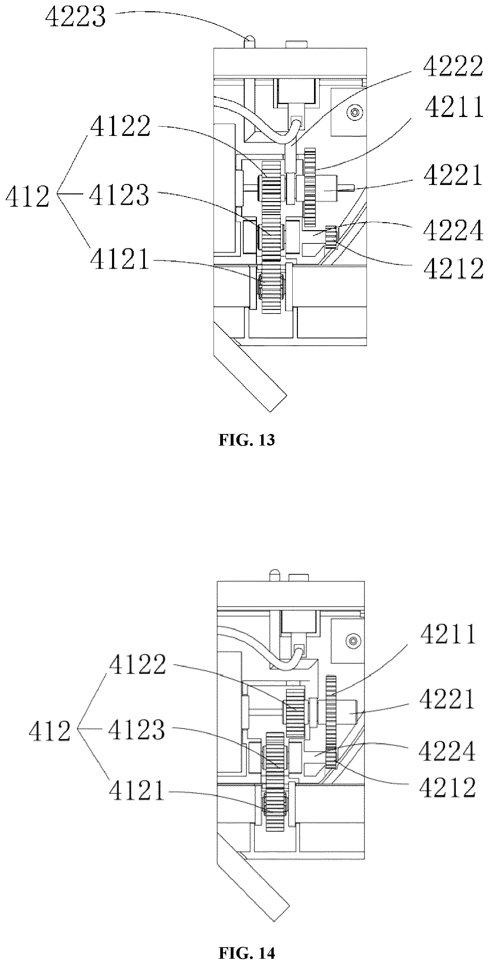

As shown in FIG. 13, the adjustment transmission assembly 421 includes a fourth transmission piece 4211 coaxially disposed with the second transmission piece 4122, and a fifth transmission piece 4212 coaxially disposed with the third transmission piece 4123; the shifting assembly 422 includes a driving wheel shaft 4221 connected to an output shaft of the power source 411, and a shifter lever 4222 connected to the driving wheel shaft 4221; the third transmission piece 4123 and the fifth transmission piece 4212 are both disposed on the driven wheel shaft 4224; the driving wheel shaft 4221 and the output shaft are slidably connected and rotate synchronously; and the shifter lever 4222 is used for driving the second transmission piece 4122 to be connected to the third transmission piece 4123 or for driving the fourth transmission piece 4211 to be connected to the fifth transmission piece 4212.

In one embodiment, all the transmission pieces are gears. The power source 411 is the motor; the first transmission piece 4121 is an output gear connected to the torque shaft 210; the second transmission piece 4122 is a driving gear connected to the motor; and the third transmission piece 4123 is a transmission gear disposed on the driving shell 430. The driving gear, the transmission gear and the output gear are sequentially engaged.

In addition, in this embodiment, the adjustment transmission assembly 421 is provided with a second group of gear assemblies, i.e. the fourth transmission piece 4211 and the fifth transmission piece 4212, and the fourth transmission piece 4211 is a large speed control gear coaxially disposed with the second transmission piece 4122; and the fifth transmission piece 4212 is a small speed control gear coaxially disposed with the third transmission piece 4123.

In one embodiment, the fourth transmission piece 4211 and the second transmission piece 4122 are both disposed on the driving wheel shaft 4221; the motor directly drives the output shaft to rotate; and the driving wheel shaft 4221 is slidably connected to the output shaft of the motor, and rotates synchronously with the output shaft of the motor. The fifth transmission piece 4212 is connected to the third transmission piece 4123 by means of a driven wheel shaft 4224; and the fifth transmission piece 4212 and the third transmission piece 4123 rotate coaxially by means of the driven wheel shaft 4224.

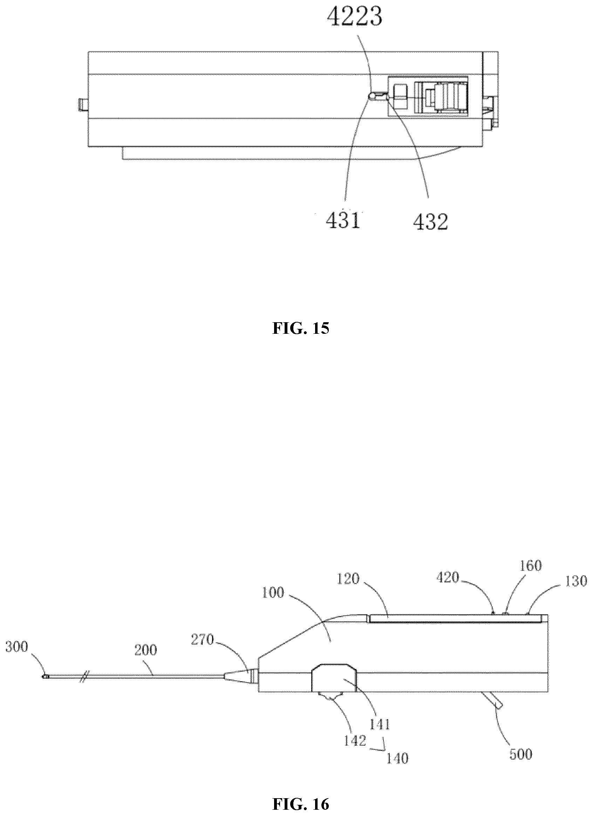

The driving wheel shaft 4221 is fixedly connected to the shifter lever 4222; and a speed control key 4223 is disposed at a tail end of the shifter lever 4222, and is slidably connected to the handle body 100. As shown in FIG. 15, a low-speed limiting groove 431 and a high-speed limiting groove 432 are formed in the driving shell 430. When the atherectomy cutter head 320 is in low-speed gear cutting, the speed control key 4223 is located in the low-speed limiting groove 431, and the second transmission piece 4122, the third transmission piece 4123 and the first transmission piece 4121 are sequentially engaged.

In this embodiment, a specific working principle of the driving module 410 is as follows: when the power source 411 works and drives the second transmission piece 4122 to rotate, that is, the motor starts to operate and drives the second transmission piece 4122 to operate, the second transmission piece 4122 is engaged with the third transmission piece 4123 to transmit a rotation speed to the third transmission piece 4123; the third transmission piece 4123 is engaged with the first transmission piece 4121 to transmit the speed to the first transmission piece 4121; the first transmission piece 4121 then transmits the speed to the torque shaft 210; and the torque shaft 210 drives the atherectomy cutter head 320 to rotate to complete low-speed gear cutting. During low-speed gear cutting, the rotation speed of the torque shaft is lower, but the torque is larger.

During switching between low-speed gear cutting and high-speed gear cutting, the speed control key 4223 is toggled to move to the high-speed limiting groove 432; the shifter lever 4222 moves under the driving of the speed control key 4223; and movement of a speed control rod drives the driving wheel shaft 4221 to move, and disengages the second transmission piece 4122 and the third transmission piece 4123. As the fourth transmission piece 4211 and the second transmission piece 4122 are both disposed on the driving wheel shaft 4221, the fourth transmission piece 4211 also moves together with the driving wheel shaft 4221, and is engaged with the fifth transmission piece 4212, so as to switch a gear engagement relationship.

In this embodiment, the number of teeth of the fourth transmission piece 4211 is larger than that of the second transmission piece 4122; and the number of teeth of the fifth transmission piece 4212 is smaller than that of the third transmission piece 4123. Therefore, by comparing an engaged transmission state of the fourth transmission piece 4211 and the fifth transmission piece 4212 with that of the second transmission piece 4122 and a driven gear, rotation speed of the fifth transmission piece 4212 is higher.

As the rotation speed of the fifth transmission piece 4212 is higher, the fifth transmission piece 4212 transmits a higher rotation speed to the third transmission piece 4123 by means of the driven wheel shaft 4224, and the third transmission piece 4123 is engaged with the first transmission piece 4121, rotation speed of the first transmission piece 4121 is higher, and then high-speed gear cutting of the atherectomy cutter head 320 is completed. During high-speed gear cutting, rotation speed of the torque shaft is higher, but torque is smaller.

Based on the embodiments, by arranging an additional gear group, i.e. the fourth transmission piece 4211 and the fifth transmission piece 4212, in the adjustment module 420, and adjusting the engagement relationship between various gear assemblies by means of the shifting assembly 422, the rotation speed of the atherectomy cutter head 320 can be adjusted between a high-speed gear and a low-speed gear, and then torque of the atherectomy cutter head 320 can be adjusted between high torque and low torque. A doctor may flexibly select the cutting speed according to the texture of the plaque, so as to avoid the cutter head jamming, improve use stability, and avoid vascular injury.

During conventional cutting, the doctor can select the high-speed gear, so as to maximize plaque resecting efficiency. In the case where the plaque is blocked or is difficult to resect, especially when the plaque or the thrombus is stuck in a bent portion of the catheter 200 due to steering of the catheter 200, the doctor can select the low-speed gear to increase torque of the torque shaft 210, thereby avoiding the phenomenon where it si difficult for the plaque or the thrombus to pass through the bent portion of the catheter 200 to ensure non-blockage of the catheter 200, avoid jamming and stopping of the atherectomy cutter head 320, and ensure smooth operation of the surgery.

In other embodiments, additional gear assemblies can further be disposed to increase a number of adjustable gears, for example, achieving three-gear adjustment, so as to provide more choices for the doctor. Besides, the above speed control solution is only an example, and any mechanical structure that can achieve torque adjustment of the torque shaft is within the scope of protection of the present embodiment.

In other embodiments, when a driving mode with an external power supply is used, output torque of the torque shaft can further be dynamically controlled by dynamically adjusting output power of the external power supply, so as to ensure use stability.

In addition, as shown in FIGS. 16 to 21, an assembly portion 110 for assembling the driving mechanism 400 is disposed on the handle body 100; the driving mechanism 400 includes the driving shell 430 for accommodating the driving module 410 and the adjustment module 420; and a disassembly connection portion 130 is disposed between the driving shell 430 and the assembly portion 110, where a sealing structure 4124 is disposed between the driving transmission assembly 412 and the handle body 100.

In this embodiment, by means of the detachable design of the driving mechanism 400, first, convenience is provided for pre-sterilization, and the power supply assembly 413 which is more difficult to sterilize in the driving mechanism 400 can be sterilized separately; and then, through quick assembly by means of the disassembly connection portion 130, difficulty of sterilization and production assembly speed of the whole resection apparatus are reduced, and production efficiency is improved. Second, after use of the resection apparatus, disassembled parts may be reused within an allowable range according to medical specifications, which increases utilization rate of the parts, and reduces use cost. In addition, after use of the resection apparatus, various parts may be quickly disassembled, and classified, recycled and scrapped flexibly according to the medical specifications, which reduces difficulty and cost of recycling.

The disassembly connection portion 130 includes a hook assembly 131 and a clamping assembly 132 which are disposed between the assembly portion 110 and the driving shell 430; the hook assembly 131 and the clamping assembly 132 are disposed at the two ends of the assembly portion 110 respectively; and a pop-up member 133 is disposed between the driving shell 430 and the bottom of the assembly portion 110.

In one embodiment, the assembly portion 110 is an assembly groove formed in the handle body 100 and used for mounting the driving mechanism 400; and the driving mechanism 400 is disposed inside the assembly portion 110, and is detachably connected to the handle body 100. Detachable replacement of the driving mechanism 400 as a whole is specifically achieved by detachably connecting the driving shell 430 to the assembly portion 110; and the driving module 410 and the adjustment module 420 are both mounted in the driving shell 430.

In this embodiment, the driving mechanism 400 is disposed into a detachable structure as a whole, so that the driving mechanism 400 maybe recycled when it meets specifications. Besides, the driving mechanism 400 and the handle body 100 may be classified and recycled according to medical use specifications.

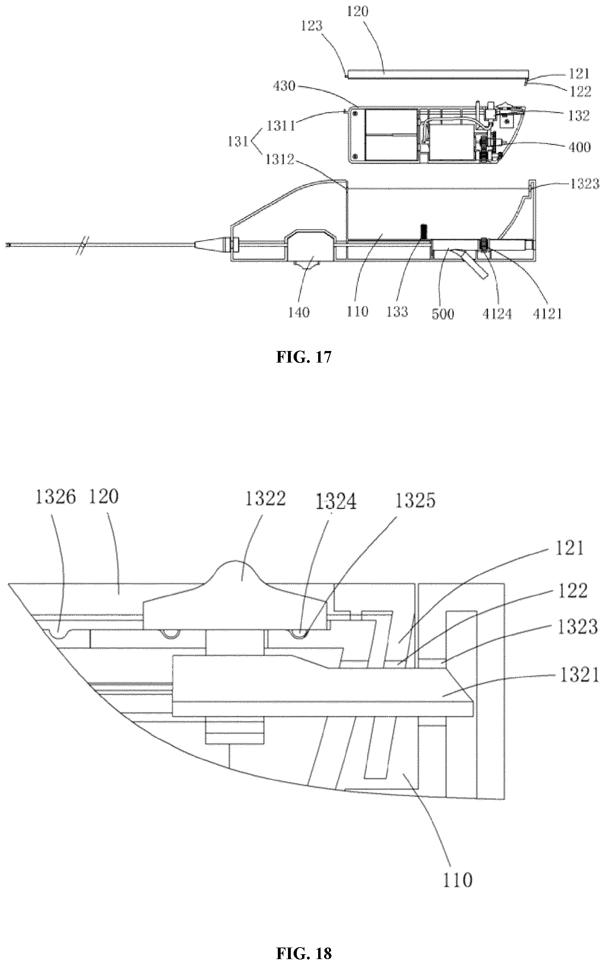

In addition, as shown in FIG. 18, the clamping assembly 132 is disposed at a proximal end of the handle body 100, and the hook assembly 131 is disposed at a distal end of the handle body 100. The clamping assembly 132 includes a locking pin 1321 slidably connected to the driving shell 430 and a locking key 1322 disposed on the locking pin 1321; and the locking key 1322 and the locking pin 1321 are integrally formed, or are bonded and fixed after being formed separately.

A locking hole 1323 being in clamping connection with the locking pin 1321 is formed in an inner wall of the assembly portion 110. A user can drive the locking pin 1321 to move by toggling the locking key 1322, and control the locking pin 1321 to be inserted into the locking hole 1323 to achieve fixation of the locking pin 1321 and the locking hole 1323, or control the locking pin 1321 to be released from insertion with the locking hole 1323 to achieve separation of the locking pin 1321 from the locking hole 1323. When the locking pin 1321 is inserted into the locking hole 1323, the driving mechanism 400 and the assembly portion 110 are in a fixed state. When the locking pin 1321 is separated from the locking hole 1323, the driving mechanism 400 and the assembly portion 110 are in a separation state.

A locking clamp 1324 is disposed at a bottom of the locking key 1322; a locking fixing groove 1325 and a separation fixing groove 1326 are formed in a surface of the driving shell 430; the separation fixing groove 1326 is formed far away from the locking hole 1323 relative to the locking fixing groove 1325; and the locking fixing groove 1325 and the separation fixing groove 1326 are used for being in clamping fixation with the locking clamp 1324 separately. When the locking key 1322 drives the locking pin 1321 to move towards a direction close to the locking hole 1323, and enables the locking clamp 1324 to be in clamping connection to an interior of the locking fixing groove 1325, the locking pin 1321 is inserted into the locking hole 1323, and the driving mechanism 400 is in clamping fixation with the assembly portion 110. When the locking key 1322 drives the locking pin 1321 to move towards a direction far away the locking hole 1323, and enables the locking clamp 1324 to be in clamping connection to the interior of the separation fixing groove 1326, the locking pin 1321 is separated from the locking hole 1323, and the driving mechanism 400 is separated from the assembly portion 110.

The pop-up member 133 for ejecting the driving mechanism 400 is disposed between the driving shell 430 and the bottom of the assembly portion 110. The pop-up member 133 is preferably a spring. That is, after the locking pin 1321 is separated from the locking hole 1323, the pop-up member 133 automatically ejects the driving mechanism 400 from the assembly portion 110, so as to facilitate the user to take out the driving mechanism 400.

The hook assembly 131 is disposed at the other end, opposite to the locking pin 1321, of the driving shell 430, and includes a hook 1311 disposed on a distal end surface of the driving shell 430 and a hook groove 1312 formed in an inner surface of the assembly portion 110 and being in hooking connection to the hook 1311.

When the driving mechanism 400 is fixed in the assembly portion 110, the hook 1311 is first in hooking connection to the hook groove 1312, so as to achieve fixation of a distal end of the driving mechanism 400 and the assembly portion 110; and then the locking key 1322 of the clamping assembly 132 is inserted into the locking hole 1323, so as to achieve fixation of the proximal end of the driving mechanism 400 and the assembly portion 110.

When the driving mechanism 400 is taken out of the assembly portion 110, the locking key 1322 of the clamping assembly 132 is first separated from the locking hole 1323, so as to separate a proximal end of the driving mechanism 400 from the assembly portion 110; and then the hook 1311 is taken out of the hook groove 1312, so as to separate the distal end of the driving mechanism 400 from the assembly portion 110.

In this embodiment, a handle upper cover 120 detachably connected to the handle body 100 and disposed on an outer side of the driving mechanism 400 is also disposed on the handle body 100.

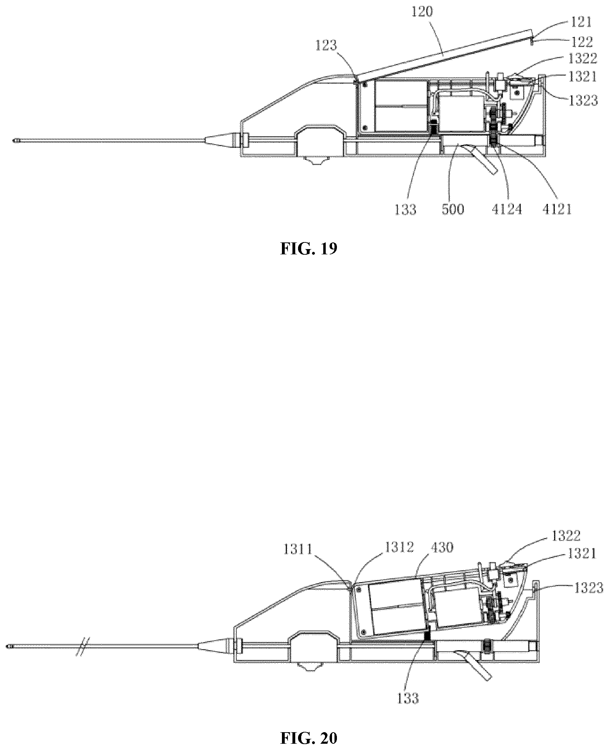

As shown in FIG. 19, a distal end of the handle upper cover 120 is fixed with the hook groove 1312 of the handle body 100 by means of a clamping structure; and an upper cover bolt 123 is disposed at the distal end of the handle upper cover 120, where the upper cover bolt 123 is connected to the hook groove 1312 in an inserting manner. An inserting plate 121 is disposed at a proximal end of the handle upper cover 120, and is disposed between the driving shell 430 and the hook groove 1312, and an inserting hole 122 matched with the locking pin 1321 is formed in the inserting plate 121. After the upper cover bolt 123 of the handle upper cover 120 is in clamping fixation with the hook groove 1312, the handle body 100 is fitted to the driving shell 430, and then the locking key 1322 is operated to move towards the locking hole 1323. The locking key 1322 first extends through the inserting hole 122 of the inserting plate 121, and then is inserted into an indentation hole, so as to achieve the purpose of fixing the handle upper cover 120 and the driving mechanism 400 on the handle body 100 at the same time.

The present embodiment protects the driving mechanism 400 by arranging the handle upper cover 120 on the driving mechanism 400, so as to prevent damage to the driving mechanism 400 due to external collision and other factors. In addition, after the handle body 100 is disassembled from the driving mechanism 400, relevant split parts may be recycled and scrapped in a classified manner according to relevant physiotherapy regulations.

In this embodiment, the steering assembly 220 is disposed at the distal end of the handle body 100, so as to facilitate operations of the doctor during the surgery. The catheter 200 extends into the handle body 100 from the distal end of the handle body 100, and is connected to the handle body 100 by means of a buffer sleeve 270. The proximal end of the catheter 200 is fixed to the discharge cavity channel 500 disposed at a middle part of the handle body 100.

The discharge cavity channel 500 is disposed at the middle part of the handle body 100; and the torque shaft 210 extends through the handle body 100. The torque shaft 210 first extends through the catheter 200, and extends into the main cavity 510 from the catheter 200. Plaque or a thrombus sent out from the catheter 200 falls into the main cavity 510, and is discharged from the waste discharge tube. The waste discharge tube is inclined relative to the main cavity 510 to facilitate discharge of the plaque or the thrombus.

The torque shaft 210 extends out of the discharge cavity channel 500, and is connected to the handle body 100 by means of a bearing. The wire guide cavity 211 of the torque shaft 210 communicates with the external space of the handle body 100, so that a guide wire extends through the torque shaft 210 by means of the wire guide cavity 211 and extends out from the distal end of the torque shaft 210, thereby establishing a path approaching the blood vessel for the catheter 200.

The driving mechanism 400 is disposed above the catheter 200 and the discharge cavity channel 500; and the driving transmission assembly 412 is disposed at a proximal end of the discharge cavity 500, and is connected to the torque shaft 210. Therefore, the plaque or the thrombus is discharged from the handle body 100 by means of the discharge cavity channel 500 disposed at the distal end of the driving transmission assembly 412, and cannot make a contact with the driving transmission assembly 412.

Besides, a sealing design is used between the driving transmission assembly 412 and the discharge cavity channel 500, that is, a sealing structure 4124 is disposed between the first transmission piece 4121 and the handle body 100. The sealing structure 4124 includes a rolling bearing fixed on the handle body 100 and a sealing ring for sealing the rolling bearing. The torque shaft 210 extends through the rolling bearing, and is fixed and in sealing connection to the rolling bearing. In one embodiment, a gap between the torque shaft 210 and the rolling bearing can be fully filled with a solder and fixed by means of welding, and then the rolling bearing and the handle body 100 can be sealed by the sealing ring. Therefore, in the embodiment, the driving mechanism 400 may be kept clean after use, so as to be conveniently recycled and may be reused.

In summary, for the present embodiment, the bending radius of the catheter 200 at the distal end is adjusted by means of the steering assembly 220, so that the catheter 200 is shifted toward a predetermined direction; the orientation of the cutter head assembly 300 is controlled, so as to reduce the risk of scraping and perforating a blood vessel, and an ideal blood vessel diameter can be obtained by means of multiple resections. Meanwhile, the adjustment module 420 for adjusting the torque of the torque shaft 210 is disposed, so that the plaque resection apparatus may more smoothly resect the plaque by adjusting the torque of the torque shaft 210 when resecting a hard plaque. In the other aspect, by arranging the driving mechanism 400 to be detachably connected to the handle body 100, the driving mechanism 400 may be recycled to reduce use the cost of the plaque resection apparatus.

### Embodiment II

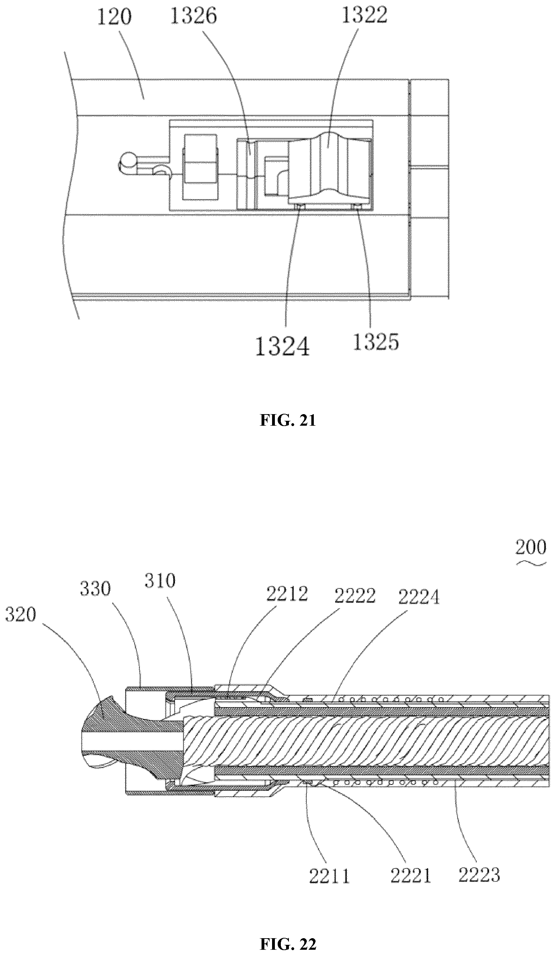

Embodiment II of the present disclosure provides a resection apparatus. As shown in FIG. 22, similarities between the embodiment II and the embodiment I are no longer repeated. The embodiment II is different from the embodiment I in that steering points 221 include a steering fixing piece 2211 disposed on a catheter 200 and a reset fixing piece 2212 disposed on a cutter head assembly 300; a steering connector 222 is used for connecting the steering fixing piece 2211 and the reset fixing piece 2212; the reset fixing piece 2212 is disposed at a proximal end of the cutter head assembly 300; and the steering fixing piece 2211 is disposed on the other side, opposite to the reset fixing piece 2212, of the catheter 200.

The steering connector 222 includes a steering connecting wire 2221 and a reset connecting wire 2222; and a steering wire cavity 2223 through which the steering connecting wire 2221 extends, and a reset wire cavity 2224 through which the reset connecting wire 2222 extends are formed in the catheter 200. The steering connecting wire 2221 is used for connecting a control assembly 140 and the steering fixing piece 2211; and the reset connecting wire 2222 is used for connecting the control assembly 140 and the reset fixing piece 2212.

The reset fixing piece 2212 is disposed at the proximal end of the cutter head assembly 300; and the steering fixing piece 2211 is disposed on the other side, opposite to the reset fixing piece 2212, of the catheter 200. The steering fixing piece 2211 is disposed on the other side, opposite to the reset fixing piece 2212, of the catheter 200, such as that the circumferential angle between the steering fixing piece 2211 and the reset fixing piece 2212 is 180 degrees.

In one embodiment, the steering wire cavity 2223 and the reset wire cavity 2224 are formed in the catheter 200; and the steering wire cavity 2223 and the reset wire cavity 2224 are both disposed in an outer tube 240 or between the outer tube 240 and a braided layer 250. One end of the steering connecting wire 2221 is connected to the steering fixing piece 2211, and then exends through the steering wire cavity 2223; and the other end is connected to the control assembly 140. One end of the reset connecting wire 2222 is connected to the steering fixing piece 2211, and then extends through the reset wire cavity 2224; and the other end is connected to the control assembly 140.

The steering connecting wire 2221 and the reset connecting wire 2222 are separately fixed on a wire takeup device 142. When a doctor rotates a control seat 141, the wire takeup device 142 slips on a sliding rod 144 relative to the control seat 141, which in turn draws the steering connecting wire 2221 and the reset connecting wire 2222 at the same time, thereby adjusting postures of the catheter 200 and the cutter head assembly 300.

The reset fixing piece 2212 is disposed on a protective sleeve 330, and is a fixed plate disposed on the protective sleeve 330; the reset connecting wire 2222 is fixed with the fixed plate by means of welding, or a connecting hole is formed in the reset fixing piece 2212; and the reset connecting wire 2222 extends through the connecting hole and is knotted and fixed. The steering fixing piece 2211 is a fixed ring disposed on the braided layer 250 of the catheter 200; the fixed ring tightly presses and fixes the steering connecting wire 2221 on the braided layer 250, or the steering connecting wire 2221 is fixed with the fixed ring by means of welding.

As the steering fixing piece 2211 is disposed on the other side, opposite to the reset fixing piece 2212, of the catheter 200, when the wire takeup device 142 draws the steering connecting wire 2221 and the reset connecting wire 2222 at the same time, the steering connecting wire 2221 controls the catheter 200 to axially displace by means of the steering fixing piece 2211; and the reset fixing piece 2212 controls the cutter head assembly 300 to radially displace by means of the reset fixing piece 2212. Under the combined action of the steering connecting wire 2221 and the reset connecting wire 2222, S-shaped steering of the cutter head assembly 300 is achieved, so that the cutter head assembly 300 and the catheter 200 keep in the same axial direction, and the risk of the cutter head assembly 300 scraping a vascular wall is reduced. The cutter head assembly 300 resects a plaque in the blood vessel before and after steering, which may make the blood vessel obtain a larger lumen.

In this embodiment, S-shaped steering of the catheter 200 and the cutter head assembly 300 is achieved by means of the steering assembly 220, so that a cutting area may be enlarged by steering the catheter 200 and the cutter head assembly 300 when the plaque in the blood vessel is cut. Meanwhile, as the cutter head assembly 300 is ensured to be the same as the catheter 200 in an axial direction under the action of the reset fixing piece 2212, during advancement of the cutter head assembly 300, the vascular wall cannot be cut by mistake, which greatly reduces the risk that the cutter head assembly 300 scrapes the vascular wall. Compared with the steering manner of the prior art, the present embodiment not only makes the blood vessel obtain a larger lumen, but also ensures safety during cutting.

### Embodiment III

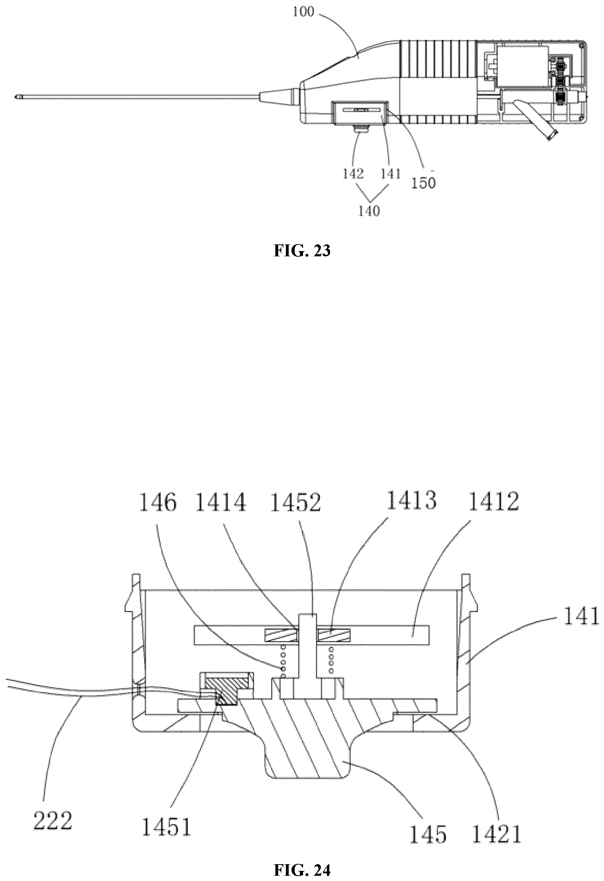

Embodiment III of the present disclosure provides a resection apparatus. As shown in FIGS. 23 and 24, similarities between the embodiment III and the embodiment I are no longer repeated. The embodiment III is different from the embodiment I in that a control groove 150 is formed in a handle body 100; a control assembly 140 is disposed inside the control groove 150; a control seat 141 is clamped in the control groove 150; a wire takeup device 142 includes a sliding knob 145 slidably connected to the control seat 141 and a wire connection point 1451 disposed on the sliding knob 145; and the wire connection point 1451 is used for connecting a steering connector 222. The steering connector 222 is a steering connecting wire 2221.

In one embodiment, a sliding groove 1412 and a slider 1413 slidably connected to the sliding groove 1412 are disposed in the control seat 141; and the sliding groove 1412 is formed in the longitudinal direction of the control seat 141. A fixing hole 1414 is formed in the slider 1413; a fixing column 1452 is disposed at one end, facing the slider 1413, of the sliding knob 145 of the control seat 141; and the fixing column 1452 extends through the fixing hole 1414, so that the sliding knob 145 is connected to the slider 1413 by means of the fixing column 1452. A control spring 146 is disposed between the sliding knob 145 and the slider 1413; the sliding knob 145 and the slider 1413 are slidably connected to the control seat 141 at the same time; and the control spring 146 has initial pressure, so that the sliding knob 145 clings to an inner wall of the control seat 141.

The locking piece 1421 is a rough contact surface disposed between the sliding knob 145 and the control seat 141. When the control assembly 140 is in a natural state, the control spring 146 drives the sliding knob 145 to cling to the inner wall of the control seat 141. As the rough contact surface is disposed between the sliding knob 145 and the control seat 141, the sliding knob 145 and the control seat 141 cannot move relative to each other under the action of friction, and the wire takeup device 142 is self-locked. When the sliding knob 145 is pressed towards inside the control seat 141, the control spring 146 is compressed due to force, and the sliding knob 145 is separated from the control seat 141. At this time, the sliding knob 145 can move relative to the control seat 141.

In other embodiments, the locking piece 1421 can further be a mutually clamped projection-groove structure that is disposed between the sliding knob 145 and the control seat 141, so as to enhance the bonding strength of the sliding knob 145 and the control seat 141 when the sliding knob 145 and the control seat 141 are fitted. In the natural state, the control spring 146 drives the sliding knob 145 to cling to the inner wall of the control seat 141, so that the wire takeup device 142 is self-locked by means of mutual clamping of the projection-groove structure.

In this embodiment, a threaded hole is formed in the sliding knob 145, and the wire connection point 1451 is a screw that is in threaded connection to the sliding knob 145. During assembly, the steering connector 222 is bonded and fixed in the threaded hole, and then the screw is screwed into the threaded hole for secondary fixation.

In specific operations, an operator presses the sliding knob 145 to separate the contacting surfaces of the sliding knob 145 and the control seat 141; the spring slides the sliding knob 145 in an axial direction of the catheter 200, which drives the steering connector 222 to move in the axial direction of the catheter 200, so that the posture of the catheter 200 is adjusted to steer the cutter head assembly 300. During a surgery, the operator does not need to press the sliding knob 145 all the time. Besides, the operator only needs one hand to achieve a steering operation on the cutter head assembly 300, which reduces the operation burden and improves operation convenience. In addition, by means of the translating adjustment, the adjustment stroke is accurate, and the steering angle maybe accurate and controllable.

### Embodiment IV

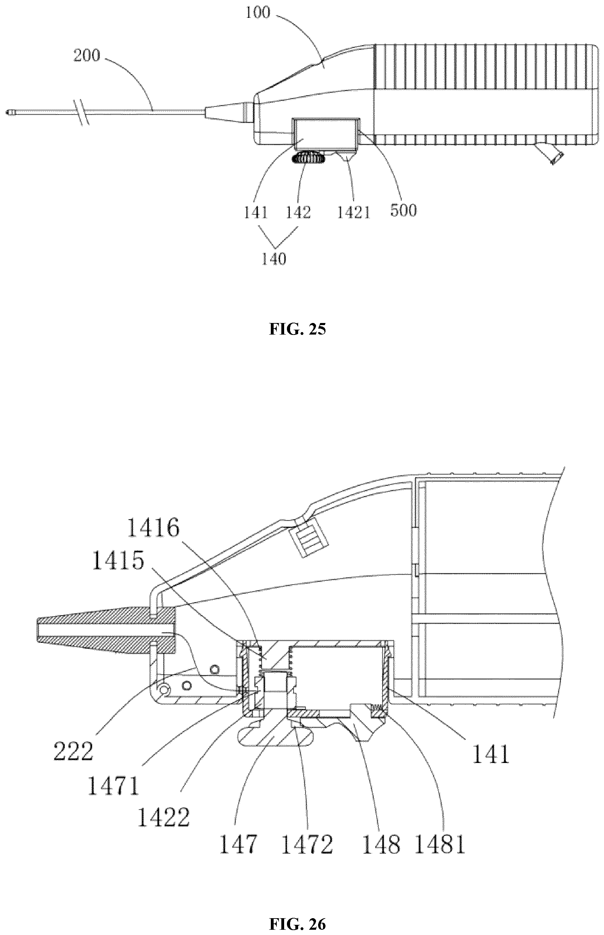

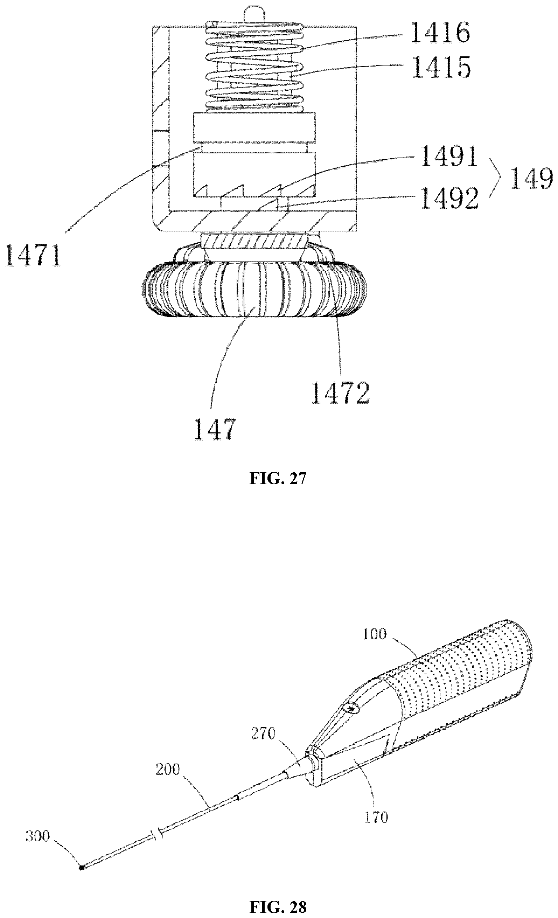

Embodiment IV of the present disclosure provides a resection apparatus. As shown in FIGS. 25 to 27, similarities between the embodiment IV and the embodiment I are no longer repeated. The embodiment IV is different from the embodiment I in that a control groove 150 is formed in a handle body 100; a control assembly 140 is disposed inside the control groove 150; a control seat 141 is clamped and fixed in the control groove 150; a wire takeup device 142 includes a rotary knob 147 rotatably connected to the control seat 141 and a locking piece 1421 used for clamping the rotary knob; the locking piece 1421 is a stop pin 148 for clamping and fixing the rotary knob 147; the stop pin 148 is slidably connected to the control seat 141; and a stop groove 1472 in clamping connection to the stop pin 148 is formed in the rotary knob 147, and a stop spring 1481 is disposed between the stop pin 148 and an inner wall of the control seat 141.

A rotary clamping block 1471 is disposed on the rotary knob 147, and is used for connecting a steering connector 222; the steering connector 222 is a steering connecting wire 2221; and the rotation of the rotary knob 147 makes the steering connector 222 wind on the rotary clamping block 1471, so as to achieve the purpose of drawing the steering connector 222 to adjust the posture of the catheter 200. In a natural state, the stop spring 1481 drives the stop pin to move towards the rotary knob 147, and is clamped in the stop groove 1472. When the rotary knob 147 needs to be rotated, the stop pin 148 is slid away from the rotary knob 147 to separate the stop pin 148 from the stop groove 1472; and at this time, the rotary knob 147 can be rotated. When the rotary knob 147 needs to be locked, the stop pin 148 is released, and the stop spring 1481 drives the stop pin 148 to slide towards the rotary knob 147, so that the stop pin 148 and the stop groove 1472 are clamped; and at this time, the rotary knob 147 cannot rotate.

In another embodiment, the control assembly 140 further includes a locking safety assembly 1422 for preventing a mis-operation; and the locking safety assembly 1422 is disposed between the control seat 141 and the wire takeup device 142.

In one embodiment, a positioning column 1415 is also disposed in the control seat 141; and a positioning spring 1416 surrounds the positioning column 1415, and is disposed between the control seat 141 and the rotary knob 147, and the rotary knob 147 can move in an own axial direction; and the locking safety assembly 1422 is disposed between the rotary clamping block 1471 and the control seat 141, where the locking safety assembly 1422 is a position clamping structure 149, where the position clamping structure 149 includes a position clamping groove 1491 formed in one end, facing the rotary knob 147, of the rotary clamping block 1471, and a position clamping block 1492 disposed at one end, facing the rotary clamping block 1471, of the control seat 141. When the position clamping block 1492 is clamped and fixed with the position clamping groove 1491, the rotary knob 147 cannot rotate.

In specific operations, in a natural state, the positioning spring 1416 pushes the rotary knob 147 outward, so that the position clamping structure 149 between the rotary clamping block 1471 and the control seat 141 is clamped and fixed. Meanwhile, the stop pin 148 and the stop groove 1472 are in a clamping state under the driving of the stop spring 1481. When the stop pin 148 is inserted into the stop groove 1472, the stop pin 148 is located between the rotary knob 147 and the control seat 141, which blocks an axial movement path of the rotary knob 147, so that the rotary knob 147 cannot be pressed, and the clamping connection between the position clamping block 1492 and the position clamping groove 1491 cannot be released either. Therefore, in the natural state, the rotary knob 147 cannot rotate. When the rotary knob 147 needs to be rotated, an operator first slides the stop pin 148 in a direction away from the rotary knob 147, so as to separate the stop pin 148 from the stop groove 1472; then, the rotary knob 147 is pressed inward, the positioning spring 1416 is compressed due to stress, and the position clamping structure 149 is separated at the same time, that is, the position clamping block 1492 is separated from the position clamping groove 1491; and at this time, the rotary knob 147 can be rotated.

Therefore, for the control assembly 140 in the embodiment, by arranging the additional locking safety assembly 1422 between the control seat 141 and the wire takeup device 142, the wire takeup device 142 is double locked, reliability of the structure is improved, and it is ensured that the posture of the cutter head assembly 300 cannot change when the doctor pushes the cutter head assembly 300 to cut plaque. When a doctor pushes the stop pin 148 and presses the rotary knob 147 at the same time, the rotary knob 147 can rotate, and the orientation of the cutter head assembly 300 cannot change due to accidental touch, which avoids an atherectomy cutter head 320 from cutting a blood vessel; and as a result, medical accidents are avoided, and a smooth operation of a surgery is ensured.

### Embodiment V

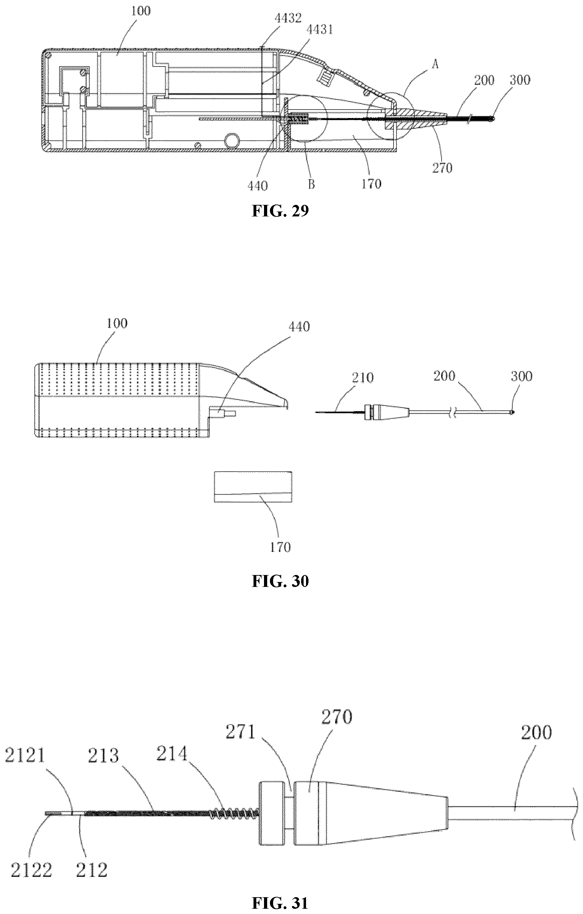

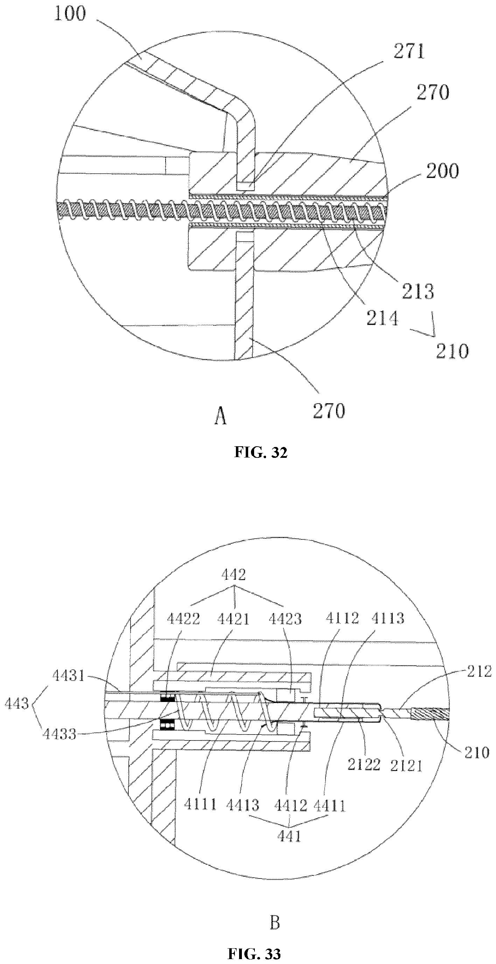

Embodiment V of the present disclosure provides a resection apparatus. As shown in FIGS. 29 to 33, similarities between the embodiment V and the embodiment I are no longer repeated. The embodiment V is different from the embodiment I in that a driving mechanism 400 is disposed inside a handle body 100, and a catheter 200 is detachably connected to the driving mechanism 400.

In one embodiment, in combination with FIG. 2, the driving mechanism 400 includes a driving module 410 disposed inside the handle body 100; the driving module 410 includes a power shaft 4111, a power source 411, and a power supply assembly 413; the power source 411 is used for driving the power shaft 4111 to rotate; a catheter 200 is detachably connected to the power shaft 4111 by means of a connection module 440, and the power source 411 is a motor; the motor is used for driving a torque shaft 210 to rotate; and the power supply assembly 413 is a battery electrically connected to the motor.

In combination with FIGS. 31 to 33, a transmission shaft 212 is disposed at a proximal end of the torque shaft 210; an inserting groove 4112 is formed in a distal end of the power shaft 4111; and the transmission shaft 212 is inserted into the inserting groove 4112, and rotates synchronously with the power shaft 4111. A clamping groove 2121 is formed in the transmission shaft 212; and the connection module 440 includes a clamping and positioning assembly 441 which is in clamping connection to the clamping groove 2121, and a clamping control assembly 442 which is used for controlling a clamping and positioning assembly 441.

In this embodiment, the torque shaft 210 includes a shaft body 213 and a wrap spring 214 disposed on the shaft body 213, and the wrap spring 214 is spirally wound and fixed on the shaft body 213, and can be fixed by means of welding; and the rotation direction of the wrap spring 214 is opposite to that of the shaft body 213. When the motor drives the torque shaft 210 to rotate, the wrap spring 214, having an opposite rotation direction to the shaft body 213, may send plaque in the catheter 200 more quickly, thereby improving resection efficiency.

In addition, at least one limiting block 2122 is disposed on the transmission shaft 212, and at least one limiting groove 4113 is formed in an inner wall of the inserting groove 4112. After the transmission shaft 212 is inserted into the inserting groove 4112, the limiting block 2122 is aligned with the limiting groove 4113, which circumferetially fixes the transmission shaft 212 to the power shaft 4111. In one embodiment, three limiting blocks 2122 distributed in a circumferential direction are disposed on the transmission shaft 212. Each of the limiting blocks 2122 is inserted into each of the limiting grooves 4113, which makes the power shaft 4111 and the transmission shaft 212 rotate synchronously in a circumferential direction, so that the torque shaft 210 rotates synchronously with the power shaft 4111.

In addition, the clamping and positioning assembly 441 includes a clamping piece 4411, a positioning piece 4412 and a connection end portion 4413, and the clamping piece 4411 is disposed on the power shaft 4111 and is used for being in clamping connection to the clamping groove 2121; the positioning piece 4412 is movably connected to the power shaft 4111; the positioning piece 4412 is used for positioning the clamping piece 4411; the connection end portion 4413 is used for connecting a clamping control piece 443 and the clamping piece 4411; and the clamping control assembly 442 controls the clamping piece 4411 to be in clamping connection to or separated from the clamping groove 2121 by controlling the connection end portion 4413.

When the transmission shaft 212 is inserted into a bottom of the inserting groove 4112, a head of the clamping piece 4411 is in clamping connection to the clamping groove 2121. As the clamping piece 4411 is axially fixed with the power shaft 4111 by means of the positioning piece 4412, axial displacements of the transmission shaft 212 and the power shaft 4111 are limited when the clamping piece 4411 is in clamping connection to the clamping groove 2121.

In this embodiment, the clamping piece 4411 is a position clamping elastic sheet with a head bent towards the transmission shaft 212, and the positioning piece 4412 is a fixing bolt in threaded connection to the power shaft 4111. A through-hole through which the positioning piece 4412 extends is formed in the clamping piece 4411. The positioning piece 4412 extends through the through-hole in the clamping piece 4411 and is in threaded connection to the power shaft 4111, so that the clamping piece 4411 is axially fixed to the power shaft 4111, and can rotate with the positioning piece 4412 as a center point to be in clamping connection to the clamping groove 2121 or separated from the clamping groove 2121.

The connection end portion 4413 is integrally formed on the clamping piece 4411, and is disposed at a tail end of the clamping piece 4411. The connection end portion 4413 is a driving elastic sheet, and the driving elastic sheet bends in a direction deviating from the power shaft 4111. When the connection end portion 4413 is squeezed towards the power shaft 4111, the clamping piece 4411 rotates with the positioning piece 4412 as a midpoint, so that the head of the clamping piece 4411 is separated from the clamping groove 2121, separating the power shaft 4111 from the transmission shaft 212 in an axial direction.

The clamping control assembly 442 includes a shaft sleeve 4421 disposed on the handle body 100, a fixed end portion 4422 fixedly disposed on an inner side of the shaft sleeve 4421, and a moving end portion 4423 movably connected to the shaft sleeve 4421 and the clamping control piece 443. The moving end portion 4423 is disposed between the positioning piece 4412 and the connection end portion 4413. The clamping control piece 443 is used for controlling the moving end portion 4423 to move. The power shaft 4111 extends through the fixed end portion 4422 and the moving end portion 4423 and then is connected to the transmission shaft 212.

The fixed end portion 4422 is a fixed bearing connected to the power shaft 4111, and the moving end portion 4423 is a sliding press block slidably connected to the shaft sleeve 4421 in an axial direction of the shaft sleeve 4421. The clamping control piece 443 includes an elastic piece 4433 connected to the fixed end portion 4422 and the moving end portion 4423, and a pull rope 4431 for controlling a position of the sliding press block.

A pull ring 4432 is disposed at a tail end of the pull rope 4431; the connection end portion 4413 is integrally connected to the clamping piece 4411; a proximal end of the connection end portion 4413 is warped; and the moving end portion 4423 slips between the connection end portion 4413 and the clamping piece 4411.

In one embodiment, the fixed end portion 4422 is fixedly connected to an inner side of the shaft sleeve 4421 and connected to the power shaft 4111; and the power shaft 4111 rotates with the positioning piece 4412 as a base point. The elastic piece 4433 is disposed between the moving end portion 4423 and the positioning piece 4412, and is used for driving the moving end portion 4423 to slip in a direction away from the positioning piece 4412; the pull rope 4431 is disposed at one end that is close to the moving end portion 4423 of the elastic piece 4433 or is disposed at the moving end portion 4423; and one end of the pull rope 4431 is connected to the elastic piece 4433 or the moving end portion 4423, and the other end of the pull rope 4431 is connected to the pull ring 4432 on an outer side of the handle body 100. An operator can draw the pull rope 4431 by means of the pull ring 4432, to make the moving end portion 4423 move towards the fixed end portion 4422, and the elastic piece 4433 is a spring, the pull rope 4431 is a nickel-titanium wire, and the nickel-titanium wire is welded to the spring.

In an initial state, the moving end portion 4423 is located on one side away from the fixed bearing under applied force of the elastic piece 4433; and at this time, the moving end portion 4423 is located on the clamping piece 4411, and the head of the clamping piece 4411 is in clamping connection to the interior of the clamping groove 2121. When the operator draws the pull rope 4431, the moving end portion 4423 moves towards the fixed end portion 4422 in an axial direction of the shaft sleeve 4421, and squeezes the connection end portion 4413 from two sides, so that the connection end portion 4413 moves towards the power shaft 4111, and the clamping piece 4411 rotates with the positioning piece 4412 as the center point; and as a result, the head of the clamping piece 4411 is separated from the clamping groove 2121. At this time, the power shaft 4111 may be axially separated from the transmission shaft 212.

From the above, in this embodiment, by arranging the clamping and positioning assembly 441 and the clamping control assembly 442, the torque shaft 210 may be axially and detachably connected to the power shaft 4111; and by means of cooperation of the limiting block 2122 and the limiting groove 4113, the torque shaft 210 and the power shaft 4111 coaxially rotate, and the motor outputs torque and drives the torque shaft 210 by means of the power shaft 4111 to rotate.

A collection portion 170 is disposed on the handle body 100, covers an outer side of a proximal end of the catheter 200, and is used for collecting plaque discharged from the catheter 200. Besides, the collection portion 170 is detachably connected to the handle body 100; and the proximal end of the catheter 200 is detachably connected to the collection portion 170. A buffer sleeve 270 is disposed at the proximal end of the catheter 200; a sealing groove 271 is formed in the buffer sleeve 270; and the handle body 100 and the collection portion 170 are separately connected to the catheter 200 by means of the sealing groove 271.

In this embodiment, the collection portion 170 is disposed at a bottom of a distal end of the handle body 100; a collection cavity is formed between the collection portion 170 and the handle body 100; and the resected plaque is accommodated by the collection cavity. The proximal end of the catheter 200 is fixedly connected to a distal end of a buffer catheter 200, or the proximal end of the catheter 200 is fixedly connected to the proximal end of the buffer catheter 200. The torque shaft 210 sequentially extends through the catheter 200 and the buffer catheter 200, and extends into the collection portion 170. The plaque or the thrombus washed out from the catheter 200 falls into the collection portion 170.

The shaft sleeve 4421 is fixedly connected to the handle body 100, and protrudes towards the collection portion 170; and other components of the clamping control assembly 442 are all disposed in an inner cavity of the shaft sleeve 4421. One end of the power shaft 4111 is connected to the power source 411, and the other end of the power shaft 4111 extends out of the shaft sleeve 4421 and is connected to the transmission shaft 212.

As the proximal end of the catheter 200 is connected to the buffer sleeve 270, the plaque or the thrombus in the catheter 200 may fall into the collection portion 170 due to high-speed rotation of the torque shaft 210 and its own gravity force after passing through the buffer sleeve 270, and cannot continue to move forward along with the torque shaft 210. Therefore, the clamping control assembly 442 disposed inside the shaft sleeve 4421 is protected.

In addition , as shown in FIG. 30, in this embodiment, the length of the shaft body 213 is larger than that of a warp spring 214; and the wrap spring 214 is not disposed on the shaft body 213 located in an interval segment between a proximal end of the wrap spring 214 and the transmission shaft 212. Through the above solution, after the plaque or the thrombus discharged from the catheter 200 is moved to the proximal end of the wrap spring 214, it will fall into the collection portion 170 due to high-speed rotation of the torque shaft 210 and its own gravity force, thereby ensuring that the plaque or the thrombus cannot continue to move forward to affect the clamping control assembly 442.

In one embodiment, the buffer sleeve 270 is fixedly disposed on the catheter 200, and is made of silica gel; and the sealing groove 271 is formed in the buffer sleeve 270. The sealing groove 271 is sunkened towards the interior of the buffer sleeve 270 and wraps around the buffer sleeve 270, forming a circular groove. After the buffer sleeve 270 is combined with the handle body 100 and the collection portion 170, edges of the handle body 100 and the collection portion 170 are connected by means of a buffer groove to realize sealing.

Through the above technical solution of the embodiment, the doctor can replace the catheter 200 according to actual use. In specific operations, the collection portion 170 is removed first, and then the pull ring 4432 on the handle body 100 is pulled. Pull force is transmitted to the elastic piece 4433 along the pull rope 4431 by means of the pull ring 4432, so that the moving end portion 4423 moves towards the fixed end portion 4422. During movement of the moving end portion 4423, the moving end portion 4423 gradually over-presses the connection end portion 4413, so that the warped connection end portion 4413 moves towards the power shaft 4111. Under a linkage action of the connection end portion 4413, the clamping piece 4411 embedded into the clamping groove 2121 is gradually separated from the clamping groove 2121. When the clamping piece 4411 is wholly separated from the interior of the clamping groove 2121, the operator can disassemble the torque shaft 210 from the power shaft 4111 in an axial direction.

When a new torque shaft 210 is charged, the pull ring 4432 is first drawn, then the torque shaft 210 is inserted into the inserting groove 4112, and then the pull ring 4432 is released. After it is confirmed that the clamping piece 4411 is clamped into the clamping groove 2121, the collection portion 170 is mounted on the handle body 100.

This embodiment includes the design of the replaceable catheter 200, which improves flexibility and economy of a surgery, and avoids the plaque or the thrombus being unable to be discharged from the blood vessel in time due to blockage of the catheter 200 to result in distal embolism, thereby ensuring life safety of a patient.

The above are only the preferred specific embodiments of the present disclosure, but the scope of protection of the present disclosure is not limited to this. Any changes or replacements that can be easily thought of by anyone familiar with the technical field within the scope of the disclosed technology should be covered within the scope of protection of the present disclosure. Therefore, the protection scope of the present disclosure should be based on the protection scope of the claims.

## Claims

1. A resection apparatus, comprising a handle body, a catheter communicating with the handle body, and a cutter head assembly disposed at a distal end of the catheter; wherein a steering assembly disposed on the catheter comprises a first steering point disposed on the catheter, a second steering point disposed on the catheter, and a steering connector for connecting the first steering point and the second steering point; wherein a control assembly for controlling the steering assembly is disposed on the handle body.

2. The resection apparatus according to claim 1, wherein the steering connector is fixedly connected to the first steering point, and movablely connected to the second steering point; the distance between the first steering point and the distal end of the catheter is smaller than that between the second steering point and the distal end of the catheter; and the first steering point is disposed on the other side opposite to the second steering point.

3. The resection apparatus according to claim 2, wherein the steering connector comprises a steering control wire; a control wire cavity through which the steering control wire extends is formed in the catheter; the second steering point is disposed at a distal end of the control wire cavity; and the steering control wire extends through the second steering point, winds around the catheter, and then is fixedly connected to the first steering point.

4. The resection apparatus according to claim 3, wherein the control assembly comprises a control seat disposed on the handle body and a wire takeup device movably connected to the control seat; and the wire takeup device is connected to the steering control wire and used for drawing the steering control wire to adjust the posture of the catheter.

5. The resection apparatus according to claim 1, wherein a torque shaft connected to the cutter head assembly is disposed in the catheter, and a driving mechanism for driving the torque shaft to rotate is disposed in the handle body.

6. The resection apparatus according to claim 5, wherein the cutter head assembly comprises a limiting seat connected to the catheter and an atherectomy cutter head disposed on the limiting seat; the atherectomy cutter head is fixedly connected to one end of the torque shaft; the torque shaft drives the atherectomy cutter head to be rotatably connected to the limiting seat; and a protective sleeve covering a side surface of the atherectomy cutter head is disposed on the limiting seat.

7. The resection apparatus according to claim 5, wherein the catheter comprises an inner tube and an outer tube disposed on an outer side of the inner tube; the inner tube surrounds an outer side of the torque shaft; the torque shaft and the inner tube are in clearance fit; and a braided layer is disposed between the inner tube and the outer tube.

8. The resection apparatus according to claim 5, wherein a discharge cavity channel connected to the catheter is formed in the handle body, and communicates with the catheter and external space of the handle body.

9. The resection apparatus according to claim 8, wherein the torque shaft extends through the discharge cavity channel, and is connected to the driving mechanism; and the driving mechanism comprises a power source, a driving transmission assembly for connecting the power source and the torque shaft, and a power supply electrically connected to the power source.

10. The resection apparatus according to claim 8, wherein the discharge cavity channel comprises a main cavity communicating with the catheter and a waste discharge tube disposed on a side surface of the main cavity; and the torque shaft extends through the discharge cavity channel.

11. A resection apparatus, comprising a handle body, a catheter communicating with the handle body, and a cutter head assembly disposed at a distal end of the catheter, wherein the resection apparatus further comprises a steering assembly and a control assembly, the steering assembly comprises a steering fixing piece disposed on the catheter, a reset fixing piece disposed on the cutter head assembly and a steering connector for connecting the steering fixing piece and the reset fixing piece; wherein a control assembly for controlling the steering connector is disposed on the handle body.

12. The resection apparatus according to claim 11, wherein the reset fixing piece is disposed at a proximal end of the cutter head assembly; and the steering fixing piece is disposed on the other side, opposite to the reset fixing piece, of the catheter.

13. The resection apparatus according to claim 12, wherein the steering connector comprises a steering connecting wire and a reset connecting wire; a steering wire cavity through which the steering connecting wire extends and a reset wire cavity through which the reset connecting wire extends are disposed on the catheter; the steering connecting wire is used for connecting the control assembly and the steering fixing piece; and the reset connecting wire is used for connecting the control assembly and the reset fixing piece.

14. The resection apparatus according to claim 13, wherein the control assembly comprises a control seat disposed on the handle body and a wire takeup device movably connected to the control seat; and the wire takeup device is connected to the steering connecting wire and the reset connecting wire, and is used for drawing the steering connecting wire and the reset connecting wire to adjust the postures of the catheter and the cutter head assembly.

15. The resection apparatus according to claim 11, wherein a torque shaft connected to the cutter head assembly is disposed in the catheter, a driving mechanism for driving the torque shaft to rotate is disposed in the handle body, and a spring tube for enhancing the torque resistance performance of the catheter is embedded inside the distal end of the catheter.

16. The resection apparatus according to claim 15, wherein the cutter head assembly comprises a limiting seat connected to the catheter and an atherectomy cutter head disposed on the limiting seat; the atherectomy cutter head is fixedly connected to one end of the torque shaft; the torque shaft drives the atherectomy cutter head to be rotatably connected to the limiting seat; a protective sleeve covering a side surface of the atherectomy cutter head is disposed on the limiting seat; a cutter head clamping groove is formed in the atherectomy cutter head; and a limiting boss in clamping fit to the cutter head clamping groove is disposed on the limiting seat.

17. The resection apparatus according to claim 15, wherein the catheter comprises an inner tube and an outer tube disposed on an outer side of the inner tube; the inner tube surrounds an outer side of the torque shaft; the torque shaft and the inner tube are in clearance fit; a braided layer is disposed between the inner tube and the outer tube; and the spring tube surrounds an outer side of the braided layer, or the braided layer surrounds an outer side of the spring tube, and the spring tube is fixedly connected to the braided layer.

18. The resection apparatus according to claim 15, wherein a discharge cavity channel connected to the catheter is formed in the handle body, and communicates with the catheter and external space of the handle body.

19. The resection apparatus according to claim 18, wherein the torque shaft extends through the discharge cavity channel, and is connected to the driving mechanism; and the driving mechanism comprises a power source, a driving transmission assembly for connecting the power source and the torque shaft, and a power supply electrically connected to the power source.

20. The resection apparatus according to claim 19, wherein the discharge cavity channel comprises a main cavity communicating with the catheter and a waste discharge tube disposed on a side surface of the main cavity; and the torque shaft extends through the discharge cavity channel.

21. A resection apparatus, comprising a handle body, a catheter communicating with the handle body, and a cutter head assembly disposed at a distal end of the catheter, wherein a torque shaft connected to the cutter head assembly is disposed in the catheter, a steering assembly for steering a distal end portion of the catheter is disposed on the catheter, a control assembly for controlling the steering assembly is disposed on the handle body, the control assembly comprises a control seat disposed on the handle body and a wire takeup device movably connected to the control seat; and the wire takeup device comprises a locking piece for self locking.

22. The resection apparatus according to claim 21, wherein the control assembly further comprises a locking safety assembly for preventing a misoperation; and the locking safety assembly is disposed inside the control seat and located between the control seat and the wire takeup device.

23. The resection apparatus according to claim 21, wherein the steering assembly comprises at least two steering points and a steering connector for connecting the steering points; and the wire takeup device is used for connecting the steering connector and drawing the steering connector to adjust a posture of the catheter and/or the cutter head assembly.

24. The resection apparatus according to claim 23, wherein the steering points comprise a steering fixing piece disposed on the catheter and a reset fixing piece disposed on the cutter head assembly; the steering connector is used for connecting the steering fixing piece and the reset fixing piece; the reset fixing piece is disposed at a proximal end of the cutter head assembly; and the steering fixing piece is disposed on the other side, opposite to the reset fixing piece, of the catheter.

25. The resection apparatus according to claim 24, wherein the steering connector comprises a steering connecting wire and a reset connecting wire; a steering wire cavity through which the steering connecting wire penetrates and a reset wire cavity through which the reset connecting wire penetrates are disposed on the catheter; the steering connecting wire is used for connecting the control assembly and the steering fixing piece; and the reset connecting wire is used for connecting the control assembly and the reset fixing piece.

26. The resection apparatus according to claim 23, wherein the steering points comprise a first steering point and a second steering point which are disposed on the catheter, the steering connector is fixedly connected to the first steering point, and movablely connected to the second steering point; a distance between the first steering point and the distal end of the catheter is smaller than that between the second steering point and the distal end of the catheter; and the first steering point is disposed on the other side opposite to the second steering point.

27. The resection apparatus according to claim 26, wherein the steering connector comprises a steering control wire; a control wire cavity through which the steering control wire penetrates is formed in the catheter; the second steering point is disposed at a distal end of the control wire cavity; and the steering control wire penetrates through the second steering point, rotates around the catheter, and then is fixedly connected to the first steering point.

28. The resection apparatus according to claim 21, wherein the cutter head assembly comprises a limiting seat connected to the catheter and an atherectomy cutter head disposed on the limiting seat; the atherectomy cutter head is fixedly connected to one end of the torque shaft; the torque shaft drives the atherectomy cutter head to be rotatably connected to the limiting seat; and a protective sleeve covering a side surface of the atherectomy cutter head is disposed on the limiting seat.

29. The resection apparatus according to claim 21, wherein the catheter comprises an inner tube and an outer tube disposed on an outer side of the inner tube; the inner tube sleeves an outer side of the torque shaft; the torque shaft and the inner tube are in clearance fit; and a braided layer is disposed between the inner tube and the outer tube.

30. The resection apparatus according to claim 1, wherein a discharge cavity channel connected to the catheter is formed in the handle body, and communicates with the catheter and external space of the handle body; the torque shaft penetrates through the discharge cavity channel and is connected to a driving mechanism; the discharge cavity channel comprises a main cavity communicating with the catheter, and a waste discharge tube disposed on a side surface of the main cavity; the torque shaft penetrates through the main cavity, and communicates with the external space of the handle body; and a wire guide cavity running through the torque shaft is disposed inside the torque shaft.

31. A resection apparatus, comprising a handle body, a catheter communicating with the handle body, and a cutter head assembly disposed at a distal end of the catheter, wherein a torque shaft connected to the cutter head assembly is disposed in the catheter, a steering assembly for steering a distal end portion of the catheter is disposed on the catheter, a driving mechanism is disposed on the handle body, and the driving mechanism comprises a driving module for driving the torque shaft to rotate, and an adjustment module for controlling the driving module to adjust torque of the torque shaft.

32. The resection apparatus according to claim 31, wherein the driving module comprises a power source, and a driving transmission assembly for connecting the power source and the torque shaft, the adjustment module comprises an adjustment transmission assembly connected to the driving transmission assembly, and a shifting assembly; and the shifting assembly is used for controlling fitting relationship between the adjustment transmission assembly and the driving transmission assembly, to adjust the torque of the torque shaft.

33. The resection apparatus according to claim 32, wherein the driving transmission assembly comprises a first transmission piece connected to the torque shaft, a second transmission piece connected to the power source, and a third transmission piece disposed on a driving shell, and the third transmission piece is connected between the first transmission piece and the second transmission piece.

34. The resection apparatus according to claim 33, wherein the adjustment transmission assembly comprises a fourth transmission piece coaxially disposed with the first transmission piece, and a fifth transmission piece coaxially disposed with the third transmission piece; the shifting assembly comprises a driving wheel shaft connected to an output shaft of the power source, and a shifter lever connected to the driving wheel shaft; the third transmission piece and the fifth transmission piece are both disposed on the driving wheel shaft; the driving wheel shaft and the output shaft are slidably connected and rotate synchronously; and the shifter lever is used for driving the second transmission piece to be connected to the third transmission piece or driving the fourth transmission piece to be connected to the fifth transmission piece.

35. The resection apparatus according to claim 32, wherein a power supply assembly for driving the power source is disposed in a driving shell, or a power supply assembly for connecting an external power supply and electrically connected to the power source is disposed on the driving shell.

36. The resection apparatus according to claim 31, wherein the driving mechanism is detachably connected to the handle body, an assembly portion for assembling the driving mechanism and a handle upper cover detachably connected to the handle body and disposed on an outer side of the driving mechanism are disposed on the handle body, the driving mechanism comprises a driving shell for accommodating the driving module and the adjustment module, and a disassembly connection portion is disposed between the driving shell and an assembly groove.

37. The resection apparatus according to claim 31, wherein a control assembly for controlling the steering assembly is disposed on the handle body, and the steering assembly comprises at least two steering points and a steering connector for connecting the steering points; the control assembly comprises a control seat disposed on the handle body and a wire takeup device movably connected to the control seat; and the wire takeup device is used for connecting the steering connector and drawing the steering connector to adjust a posture of the catheter and/or the cutter head assembly.

38. The resection apparatus according to claim 31, wherein the cutter head assembly comprises a limiting seat connected to the catheter and an atherectomy cutter head disposed on the limiting seat; the atherectomy cutter head is fixedly connected to one end of the torque shaft; the torque shaft drives the atherectomy cutter head to be rotatably connected to the limiting seat; and a protective sleeve covering a side surface of the atherectomy cutter head is disposed on the limiting seat.

39. The resection apparatus according to claim 31, wherein the catheter comprises an inner tube and an outer tube disposed on an outer side of the inner tube; the inner tube sleeves an outer side of the torque shaft; the torque shaft and the inner tube are in clearance fit; and a braided layer is disposed between the inner tube and the outer tube.

40. The resection apparatus according to claim 31, wherein a discharge cavity channel connected to the catheter is formed in the handle body, and communicates with the catheter and external space of the handle body; the torque shaft penetrates through the discharge cavity channel and is connected to the driving mechanism; the discharge cavity channel comprises a main cavity communicating with the catheter, and a waste discharge tube disposed on a side surface of the main cavity; the torque shaft penetrates through the main cavity, and communicates with the external space of the handle body; and a wire guide cavity running through the torque shaft is disposed inside the torque shaft.

41. A resection apparatus, comprising a handle body, a catheter communicating with the handle body, and a cutter head assembly disposed at a distal end of the catheter, wherein a torque shaft connected to the cutter head assembly is disposed in the catheter, a driving mechanism for driving the torque shaft to rotate, and an assembly portion for assembling the driving mechanism are disposed on the handle body, and the driving mechanism is detachably connected to the assembly portion.

42. The resection apparatus according to claim 41, wherein the driving mechanism comprises a driving shell detachably connected to the assembly portion and a driving module disposed in the driving shell, and a disassembly connection portion is disposed between the driving shell and the assembly portion.

43. The resection apparatus according to claim 42, wherein the disassembly connection portion comprises a hook assembly and a clamping assembly which are disposed between the assembly portion and the driving shell; the hook assembly and the clamping assembly are disposed at two ends of the assembly portion respectively; and a pop-up member is disposed between the driving shell and a bottom of the assembly portion.

44. The resection apparatus according to claim 43, wherein a handle upper cover detachably connected to the handle body and disposed on an outer side of the driving mechanism is disposed on the handle body, the driving module comprises a power source for driving the torque shaft to rotate and a driving transmission assembly for connecting the power source and the torque shaft, and a sealing structure is disposed between the driving transmission assembly and the handle body.

45. The resection apparatus according to claim 44, wherein the driving transmission assembly comprises a first transmission piece and a second transmission piece connected to the first transmission piece, the power source drives the second transmission piece to connect, the first transmission piece is used for driving the torque shaft to rotate, and the sealing structure is disposed between the first transmission piece and the handle body.

46. The resection apparatus according to claim 42, wherein the driving module further comprises a power supply assembly for driving a power source, or a power supply assembly for connecting an external power supply and electrically connected to the power source is disposed on the driving shell.

47. The resection apparatus according to claim 42, wherein the driving mechanism further comprises an adjustment module for controlling the driving module to adjust torque of the torque shaft.

48. The resection apparatus according to claim 41, wherein the cutter head assembly comprises a limiting seat connected to the catheter and an atherectomy cutter head disposed on the limiting seat; the atherectomy cutter head is fixedly connected to one end of the torque shaft; the torque shaft drives the atherectomy cutter head to be rotatably connected to the limiting seat; and a protective sleeve covering a side surface of the atherectomy cutter head is disposed on the limiting seat.

49. The resection apparatus according to claim 41, wherein the catheter comprises an inner tube and an outer tube disposed on an outer side of the inner tube; the inner tube sleeves an outer side of the torque shaft; the torque shaft and the inner tube are in clearance fit; and a braided layer is disposed between the inner tube and the outer tube.

50. The resection apparatus according to claim 41, wherein a discharge cavity channel connected to the catheter is formed in the handle body, and communicates with the catheter and external space of the handle body; the torque shaft penetrates through the discharge cavity channel and is connected to the driving mechanism; the discharge cavity channel comprises a main cavity communicating with the catheter, and a waste discharge tube disposed on a side surface of the main cavity; the torque shaft penetrates through the main cavity, and communicates with the external space of the handle body; and a wire guide cavity running through the torque shaft is disposed inside the torque shaft.

51. A resection apparatus, comprising a handle body, a catheter communicating with the handle body, and a cutter head assembly disposed at a distal end of the catheter, wherein a torque shaft connected to the cutter head assembly is disposed in the catheter, a driving mechanism for driving the torque shaft to rotate is disposed on the handle body, and the catheter is detachably connected to the driving mechanism.

52. The resection apparatus according to claim 51, wherein the driving mechanism comprises a driving module disposed in the handle body, and the driving module comprises a power shaft, a power source for driving the power shaft to rotate, and a power supply assembly, and the catheter is detachably connected to the power shaft through a connection module.

53. The resection apparatus according to claim 52, wherein a transmission shaft is disposed at a proximal end of the torque shaft; an inserting groove is formed in a distal end of the power shaft; the transmission shaft is inserted into the inserting groove, and rotates synchronously with the power shaft; a clamping groove is formed in the transmission shaft; and the connection module comprises a clamping and positioning assembly which is in clamping connection to the clamping groove, and a clamping control assembly for controlling the clamping and positioning assembly.

54. The resection apparatus according to claim 53, wherein the clamping and positioning assembly comprises a clamping piece disposed on the power shaft and in clamping connection to the clamping groove, a positioning piece movably connected to the power shaft and used for positioning the clamping piece, and a connection end portion connected to the clamping control assembly and the clamping piece, and the clamping control assembly controls the clamping piece to be in clamping connection to or separated from the clamping groove by controlling the connection end portion.

55. The resection apparatus according to claim 54, wherein the clamping control assembly comprises a shaft sleeve disposed on the handle body, a fixed end portion fixedly disposed on an inner side of the shaft sleeve, a moving end portion movably connected to the shaft sleeve and disposed between the positioning piece and the connection end portion and a clamping control piece for controlling the moving end portion to move, and the power shaft penetrates through the fixed end portion and the moving end portion and then is connected to the transmission shaft.

56. The resection apparatus according to claim 55, wherein the fixed end portion is a fixed bearing connected to the power shaft, the moving end portion is a slider in sliding connection to the shaft sleeve in an axial direction of the shaft sleeve, and the clamping control piece comprises an elastic piece connected to the fixed bearing and the slider, and a pull rope for controlling a position of the slider.

57. The resection apparatus according to claim 56, wherein a pull ring is disposed at a tail end of the pull rope; the connection end portion is integrally connected to the clamping piece; a proximal end of the connection end portion is warped; and the slider slips between the connection end portion and the clamping piece.

58. The resection apparatus according to claim 51, wherein a collection portion is disposed on the handle body, can be detachably connected to the handle body and covers an outer side of a proximal end of the catheter, and the proximal end of the catheter is detachably connected to the collection portion; a buffer sleeve is disposed at the proximal end of the catheter; a sealing groove is formed in the buffer sleeve; and the handle body and the collection portion are connected to the catheter by means of the sealing groove separately.

59. The resection apparatus according to claim 51, wherein the cutter head assembly comprises a limiting seat connected to the catheter and an atherectomy cutter head disposed on the limiting seat; the atherectomy cutter head is fixedly connected to one end of the torque shaft; the torque shaft drives the atherectomy cutter head to be rotatably connected to the limiting seat; and a protective sleeve covering a side surface of the atherectomy cutter head is disposed on the limiting seat.

60. The resection apparatus according to claim 51, wherein the catheter comprises an inner tube and an outer tube disposed on an outer side of the inner tube; the inner tube sleeves an outer side of the torque shaft; the torque shaft and the inner tube are in clearance fit; and a braided layer is disposed between the inner tube and the outer tube.
